# EUROPEAN PATENT APPLICATION

(11) **EP 3 706 398 A1**
(43) Date of publication of application: **09.09.2020**
(21) Application number: 17933403.2
(22) Date of filing: 20.12.2017
(51) Int. Cl.: H04M 1/725

(54) **PRESSURE MEASUREMENT METHOD AND TERMINAL**

(30) Priority: 29.11.2017 CN 201711226815
(71) Applicant: Huawei Technologies Co., Ltd., Longgang District Shenzhen, Guangdong 518129 (CN)
(72) Inventor: XU, Zhe, Shenzhen Guangdong 518129 (CN); HUANG, Jiejing, Shenzhen Guangdong 518129 (CN)
(74) Representative: Epping - Hermann - Fischer
(86) International application number: PCT/CN2017/117572
(87) International publication number: WO 2019/104779

(57) **Abstract**

This application provides a stress detection method and a terminal, and relates to the field of terminal technologies, to resolve a problem of relatively low reliability of data collected during stress detection. The method includes: obtaining an event of a terminal, determining a stress measurement time based on the event of the terminal, and detecting stress of a user based on the time. The event is at least one of a schedule, a reminder, and a running application program. This application is applicable to a stress detection process.

## Description

This application claims priority to Chinese Patent Application No. 201711226815.1, filed with the Chinese Patent Office on November 29, 2017 and entitled "STRESS DETECTION METHOD AND TERMINAL DEVICE", which is incorporated herein by reference in its entirety.

### TECHNICAL FIELD

This application relates to the field of terminal technologies, and in particular, to a stress detection method and a terminal.

### BACKGROUND

With development of communications technologies, currently, in addition to providing a basic communication function, devices such as a mobile phone and a band may further monitor health data of a user, for example, detect a heartbeat of the user, and record a quantity of walking steps of the user. To provide a better service for the user, manufacturers of the devices gradually add, to the devices, functions such as stress detection that are used to monitor a user emotion change, so as to master a user health status with reference to data collected by a sensor in daily life of the user, and provide sufficient data for medical treatment and for the user to learn of the user health status.

Currently, a stress detection device may detect user stress in real time. In a real-time stress detection manner, resources and power of the device are excessively consumed. In addition, because the user stress does not change in real time, a large quantity of invalid parameters exist in collected data. It can be learned that data collected in the foregoing stress detection process is usually of low reliability.

### SUMMARY

Embodiments of the present invention provide a stress detection method and a terminal, to resolve a problem of relatively low reliability of data collected during stress detection.

According to a first aspect, an embodiment of the present invention provides a stress detection method. The method includes: obtaining an event of a terminal, determining a stress measurement time based on the event of the terminal, and detecting stress of a user based on the time. The event is at least one of a schedule, a reminder, and a running application program.

It can be learned that the foregoing stress measurement manner can enable the stress measurement time more targeted. To be specific, for an event that may cause a change to the stress of the user, a stress measurement time can be determined based on the event, and a time point or a time period for detecting the stress of the user is determined in an occurrence process of the event. Therefore, the stress of the user is detected at the determined time point or in the determined time period. In this way, reliability of data obtained through stress detection can be effectively improved. Compared with that in a real-time stress detection manner, power consumption of the terminal is effectively reduced.

In a possible manner, the event is at least one of the schedule and the reminder. The determining a stress measurement time based on the event of the terminal, and detecting stress of a user based on the time may be specifically implemented as: determining, based on the schedule or the reminder, whether stress is caused to the user, and if the schedule or the reminder causes stress to the user, detecting the stress of the user at a time point or a time period in an occurrence process of an event in the schedule or an event in the reminder. To further reduce a probability of detecting invalid data and reduce power consumption caused to the terminal during detection, in this embodiment of the present invention, stress detection is performed at the time point or in the time period corresponding to the schedule or the reminder that may cause stress to the user.

In a possible manner, the event is the running application program. The determining a stress measurement time based on the event of the terminal, and detecting stress of a user based on the time may be specifically implemented as: determining whether the application program belongs to a first application list; if the application program belongs to the first application list, and a running time of the application program exceeds a preset time, determining whether a current heart rate of the user exceeds a resting heart rate; and detecting the stress of the user at a time point or in a time period after the current heart rate of the user exceeds the resting heart rate. The first application list is a list of application programs that affect the stress of the user. To further reduce a probability of detecting invalid data and reduce power consumption caused to the terminal during detection, in this embodiment of the present invention, stress detection is performed after an application program that may cause stress to the user is enabled.

According to a second aspect, an embodiment of the present invention provides a stress detection method. The method includes: detecting a status of a user by using a sensor, determining a stress measurement time based on the status of the user, and detecting stress of the user based on the time.

It can be learned that the foregoing stress measurement manner can enable the stress measurement time more targeted. To be specific, detection of the stress of the user is triggered only for a status of the user that may cause a change to the stress of the user. In this way, reliability of data obtained through stress detection can be effectively improved. Compared with a real-time stress detection manner, power consumption of the terminal is effectively reduced.

In a possible manner, the detecting a status of a user by using a sensor may be specifically: detecting, by using a heart rate sensor, that the user is in a sleep state, or detecting, by using a motion sensor, that the user is in a sleep state, or detecting, by using a heart rate sensor and a motion sensor, that the user is in a sleep state; detecting a physiological signal of the user by using the heart rate sensor; and determining, based on the physiological signal, whether the user is in a waking state. The determining a stress measurement time based on the status of the user, and detecting stress of the user based on the time may be specifically implemented as: when the user is in the waking state, obtaining a heart rate of the user by using the heart rate sensor; determining, based on the heart rate of the user, whether the user is in a stable state; and detecting the stress of the user at a first preset time after the user is in the stable state. Considering that after the user enters the waking state from the sleep state, the heart rate of the user changes correspondingly, and the heart rate may change slightly when the user is in the sleep state, after the user enters the waking state, a time at which the first preset time arrives may be used as a representative sampling point for stress detection. After stress detection is performed at the sampling point, changed data that is generated because the heart rate of the user that does not change for a long time is affected by the status of the user is obtained.

The first preset time may be a time point, or may be a time period.

In a possible manner, the detecting a status of a user by using a sensor may be specifically implemented as: detecting, by using a heart rate sensor, that the user is in a moving state, or detecting, by using a motion sensor, that the user is in a moving state, or detecting, by using a heart rate sensor and a motion sensor, that the user is in a moving state; detecting motion signal information of the user by using the motion sensor; and determining, based on the motion signal information, whether the user is in a stable state. The determining a stress measurement time based on the status of the user, and detecting stress of the user based on the time may be specifically implemented as: when the user is in the stable state for a preset time threshold, determining whether a current heart rate of the user is close to a resting heart rate; and detecting the stress of the user at a second preset time after the current heart rate of the user is close to the resting heart rate. Considering that the user may relax physically and mentally and release the stress after strenuous exercise or high-intensity exercise, the stress of the user may change after the user enters the stable state from the moving state. In this embodiment of the present invention, a time corresponding to a factor that may cause a change to the stress of the user is used as a collection time for stress detection.

The second preset time may be a time point, or may be a time period.

In a possible manner, the detecting a status of a user by using a sensor may be specifically implemented as: detecting motion signal information of the user by using a motion sensor; and when the motion signal information is less than a preset threshold, determining that the user is in a stable state. The determining a stress measurement time based on the status of the user, and detecting stress of the user based on the time may be specifically implemented as: when it is determined that the user is in the stable state for a preset time threshold, determining whether a current heart rate of the user is close to a resting heart rate; and detecting the stress of the user at a third preset time after the current heart rate of the user is close to the resting heart rate. Considering that the user may be anxious when the user is in a still state or the stable state for a long time, or in other words, the stress of the user may change when the user is in a specific state for a long time, in this embodiment of the present invention, if the user is in the stable state for a long time, the stress of the user may be detected at the third preset time after the current heart rate of the user is close to the resting heart rate.

The third preset time may be a time point, or may be a time period.

According to a third aspect, an embodiment of the present invention provides a stress detection apparatus. The apparatus may implement functions implemented in the embodiments in the first aspect and the possible manners. The functions may be implemented by hardware, or may be implemented by hardware by executing corresponding software. The hardware or the software includes one or more modules corresponding to the functions.

According to a fourth aspect, an embodiment of the present invention provides a stress detection apparatus. The apparatus may implement functions implemented in the embodiments in the second aspect and the possible manners. The functions may be implemented by hardware, or may be implemented by hardware by executing corresponding software. The hardware or the software includes one or more modules corresponding to the functions.

According to a fifth aspect, an embodiment of the present invention provides a terminal. A structure of the terminal includes a display, a memory, one or more processors, a plurality of application programs, and one or more programs. The one or more programs are stored in the memory. When the one or more processors execute the one or more programs stored in the memory, the terminal is enabled to implement the method according to any one of the first aspect and various possible manners of the first aspect.

According to a sixth aspect, an embodiment of the present invention provides a terminal. A structure of the terminal includes a display, a memory, and one or more processors. The one or more programs are stored in the memory. When the one or more processors execute the one or more programs, the terminal is enabled to implement the method according to any one of the second aspect and various possible manners of the second aspect.

According to a seventh aspect, an embodiment of the present invention provides a readable storage medium including an instruction. When the instruction is run on a terminal, the terminal is enabled to perform the method in any one of the first aspect and various possible manners of the first aspect.

According to an eighth aspect, an embodiment of the present invention provides a readable storage medium, including an instruction. When the instruction is run on a terminal, the terminal is enabled to perform the method in any one of the second aspect and various possible manners of the second aspect.

According to a ninth aspect, an embodiment of the present invention provides a computer program product. The computer program product includes software code, and the software code is used to perform the method in any one of the first aspect and the possible manners of the first aspect.

According to a tenth aspect, an embodiment of the present invention provides a computer program product. The computer program product includes software code, and the software code is used to perform the method in any one of the second aspect and the possible manners of the second aspect.

It may be understood that the foregoing method embodiments may be performed by a terminal, for example, a device such as a notebook computer, a smartphone, a virtual reality (Virtual Reality, VR) device, an augmented reality (Augmented Reality, AR) device, a vehicle-mounted device, or an intelligent wearable device. Certainly, the method embodiments may alternatively be performed by a wearable device and a terminal in cooperation.

According to an eleventh aspect, an embodiment of the present invention provides a system, including a terminal and a wearable device. The terminal is configured to obtain an event of the terminal, where the event is at least one of a schedule, a reminder, or a running application program, and determine a stress measurement time based on the event of the terminal; and the wearable device is configured to detect stress of a user based on the time.

In a possible manner, the terminal may determine, based on the schedule or the reminder, whether stress is caused to the user, and if the schedule or the reminder causes stress to the user, the wearable device detects the stress of the user at a time point or in a time period in an occurrence process of an event in the schedule or an event in the reminder.

In a possible manner, the terminal is configured to:
determine whether the application program belongs to a first application list, where the first application list is a list of application programs that affect the stress of the user; and if the application program belongs to the first application list and a running time of the application program exceeds a preset time, determine whether a current heart rate of the user exceeds a resting heart rate; and
the wearable device detects the stress of the user at a time point or in a time period after the current heart rate of the user exceeds the resting heart rate.

According to a twelfth aspect, an embodiment of the present invention further provides a system, including a terminal and a wearable device. The terminal or the wearable device is configured to detect a status of a user; the terminal is configured to determine a stress measurement time based on the detected status of the user; and the wearable device is configured to detect stress of the user based on the time.

In a possible manner, the wearable device is configured to detect that the user is in a sleep state, and detect a physiological signal of the user; the terminal determines, based on the physiological signal, whether the user is in a waking state; when the user is in the waking state, the wearable device obtains a heart rate of the user; the terminal determines, based on the heart rate of the user, whether the user is in a stable state; and the wearable device detects the stress of the user at a first preset time after the user is in the stable state.

In a possible manner, the wearable device is configured to detect that the user is in a sleep state, and detect motion signal information of the user; the terminal determines, based on the motion signal information, whether the user is in a stable state, and when the user is in the stable state for a preset time threshold, determines whether a current heart rate of the user is close to a resting heart rate; and the wearable device detects the stress of the user at a second preset time after the current heart rate of the user is close to the resting heart rate.

In a possible manner, the wearable device is configured to detect motion signal information of the user; the terminal is configured to: when the motion signal information is less than a preset threshold, determine that the user is in a stable state, and when it is determined that the user is in the stable state for a preset time threshold, determine whether a current heart rate of the user is close to a resting heart rate; and the wearable device detects the stress of the user at a third preset time after the current heart rate of the user is close to the resting heart rate.

In all the foregoing manners, the determining, based on the event of the terminal, the stress measurement time may be understood as: determining the stress measurement time in an occurrence process of the event. The time may be a time point in the occurrence process of the event, or the time may be a time period in the occurrence process of the event.

### BRIEF DESCRIPTION OF DRAWINGS

FIG. 1 is a first schematic structural diagram of a terminal according to an embodiment of the present invention;
FIG. 2 is a first flowchart of a stress detection method according to an embodiment of the present invention;
FIG. 3 is a schematic diagram of a scenario in which a mobile phone cooperates with a band according to an embodiment of the present invention;
FIG. 4 is a second flowchart of a stress detection method according to an embodiment of the present invention;
FIG. 5 is a third flowchart of a stress detection method according to an embodiment of the present invention;
FIG. 6 is a fourth flowchart of a stress detection method according to an embodiment of the present invention;
FIG. 7 is a fifth flowchart of a stress detection method according to an embodiment of the present invention;
FIG. 8(a) is a first schematic diagram of a display screen according to an embodiment of the present invention;
FIG. 8(b) is a second schematic diagram of a display screen according to an embodiment of the present invention;
FIG. 8(c) is a third schematic diagram of a display screen according to an embodiment of the present invention;
FIG. 8(d) is a fourth schematic diagram of a display screen according to an embodiment of the present invention;
FIG. 9 is a sixth flowchart of a stress detection method according to an embodiment of the present invention;
FIG. 10 is a seventh flowchart of a stress detection method according to an embodiment of the present invention;
FIG. 11 is an eighth flowchart of a stress detection method according to an embodiment of the present invention;
FIG. 12 is a ninth flowchart of a stress detection method according to an embodiment of the present invention;
FIG. 13 is a tenth flowchart of a stress detection method according to an embodiment of the present invention;
FIG. 14 is an eleventh flowchart of a stress detection method according to an embodiment of the present invention;
FIG. 15 is a twelfth flowchart of a stress detection method according to an embodiment of the present invention;
FIG. 16(a) is a fifth schematic diagram of a display screen according to an embodiment of the present invention;
FIG. 16(b) is a sixth schematic diagram of a display screen according to an embodiment of the present invention;
FIG. 16(c) is a seventh schematic diagram of a display screen according to an embodiment of the present invention;
FIG. 16(d) is an eighth schematic diagram of a display screen according to an embodiment of the present invention;
FIG. 17(a) is a ninth schematic diagram of a display screen according to an embodiment of the present invention;
FIG. 17(b) is a tenth schematic diagram of a display screen according to an embodiment of the present invention;
FIG. 17(c) is an eleventh schematic diagram of a display screen according to an embodiment of the present invention;
FIG. 18 is a first schematic structural diagram of a stress detection apparatus according to an embodiment of the present invention;
FIG. 19 is a second schematic structural diagram of a terminal according to an embodiment of the present invention;
FIG. 20 is a second schematic structural diagram of a stress detection apparatus according to an embodiment of the present invention; and
FIG. 21 is a third schematic structural diagram of a terminal according to an embodiment of the present invention.

### Description of reference signs:

- 201:: mobile phone
- 202:: screen of the mobile phone;
- 203:: band;
- 204:: screen of the band;
- 205:: display screen;
- 206:: basic information;
- 207:: function key; and
- 208:: page mark of the display screen.

### DESCRIPTION OF EMBODIMENTS

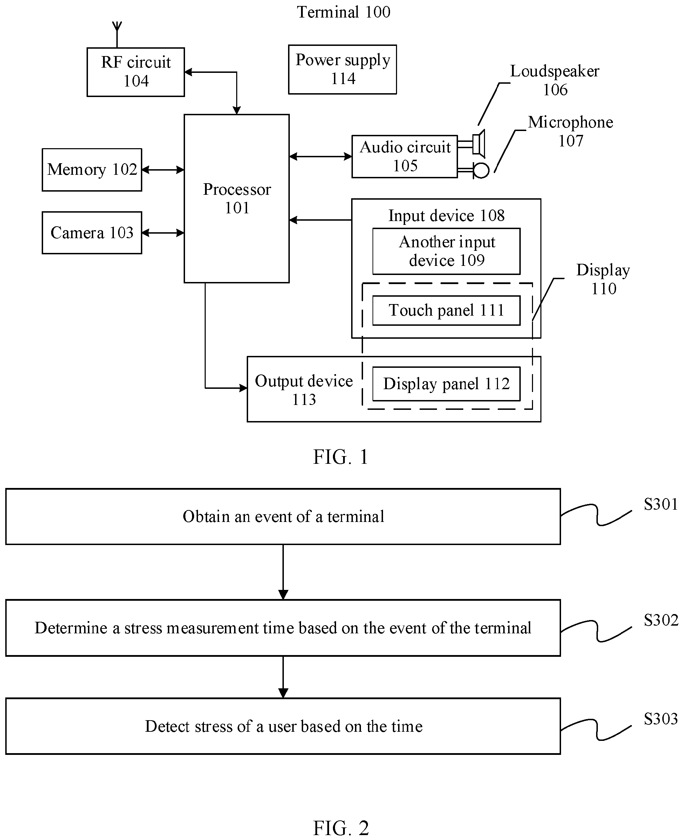

The embodiments of the present invention may be applied to a terminal. The terminal may be a device such as a notebook computer, a smartphone, a virtual reality (Virtual Reality, VR) device, an augmented reality (Augmented Reality, AR) device, a vehicle-mounted device, or an intelligent wearable device. At least a display, an input device, and a processor are disposed on the terminal. A terminal 100 is used as an example. As shown in FIG. 1, the terminal 100 includes components such as a processor 101, a memory 102, a camera 103, an RF circuit 104, an audio circuit 105, a loudspeaker 106, a microphone 107, an input device 108, another input device 109, a display 110, a touch panel 111, a display panel 112, an output device 113, and a power supply 114. The display 110 may include the touch panel 111 serving as an input device and the display panel 112 serving as an output device. It should be noted that a structure of the terminal shown in FIG. 1 constitutes no limitation on the terminal, and the terminal may include more or fewer components than those shown in the figure, or some components may be combined, or some components may be divided, or different component arrangements may be used. This is not limited herein.

The radio frequency (Radio Frequency, RF) circuit 104 may be configured to: send and/or receive information or receive and send a signal during a call. For example, if the terminal 100 is a mobile phone, the terminal 100 may send, by using the RF circuit 104 after receiving downlink information sent by a base station, the downlink information to the processor 101 for processing, and send related uplink data to the base station. The RF circuit usually includes but is not limited to an antenna, at least one amplifier, a transceiver, a coupler, a low noise amplifier (Low Noise Amplifier, LNA), a duplexer, and the like. In addition, the RF circuit 104 may further communicate with a network and another device through wireless communication. The wireless communication may use any communications standard or protocol, including but not limited to a global system for mobile communications (Global System of Mobile communication, GSM), a general packet radio service (General Packet Radio Service, GPRS), code division multiple access (Code Division Multiple Access, CDMA), wideband code division multiple access (Wideband Code Division Multiple Access, WCDMA), long term evolution (Long Term Evolution, LTE), an email, a short message service (Short Messaging Service, SMS), and the like. The memory 102 may be configured to store a software program. The processor 101 executes various function applications and data processing of the terminal 100 by running the software program stored in the memory 102. The another input device 109 may be configured to receive input digital or character information, and generate a key signal input related to user setting and function control of the terminal 100. For example, the another input device 109 may include but is not limited to one or more of a physical keyboard, a function key (such as a volume control key or an on/off key), a tracking ball, a mouse, a joystick, an optical mouse (the optical mouse is a touch-sensitive surface that does not display visual output, or an extension of a touch-sensitive surface formed by a touchscreen), or the like, or a sensor built in the terminal 100.

For example, a photoplethysmography (photoplethysmography, PPG) sensor can detect a human heart rate by using a PPG technology. An acceleration (acceleration, ACC) sensor may be configured to detect an acceleration of a user who carries, wears, or holds the terminal 100. The ACC sensor includes but is not limited to a triaxial acceleration sensor, a gyroscope sensor, and the like. An electrocardiograph may be configured to detect a change of a user's heart in each cardiac cycle in which the pacemaker, the atria, and the ventricles are successively stimulated with bioelectricity. The change is finally recorded and presented in a form of an electrocardiogram (electrocardiogram, ECG).

In the embodiments of the present invention, a pulse wave signal and a triaxial acceleration signal of the user may be obtained by using the sensor, and then filtering and extraction of a related feature such as a PPG feature point are performed, to obtain heart rate variability (heart rate variability, HRV) related parameters, for example, features such as a heart rate, root mean square of successive differences (root mean square of successive differences, RMSSD) RMSSD between two adjacent R waves, a standard deviation of NN intervals (standard diviation of NN intervals, SDNN), a proportion of the number of pairs of successive NNs that differ by more than 50 ms divided by the total number of NNs (PNN50), a low frequency (low frequency, LF) power, and a high frequency (high frequency, HF) power. Then, the processor analyzes and calculates the HRV related parameters, to obtain a stress value of the user. An RRinterval (RRI) is an interval between two consecutive heart rate waveforms, and the heart rate waveform includes but is not limited to signals such as an ECG and a PPG.

The display 110 may be configured to display information entered by the user or information provided for the user, and various menus of the terminal 100, and may further receive a user input. In addition, the display panel 112 may be configured in a form of a liquid crystal display (Liquid Crystal Display, LCD), an organic light-emitting diode (Organic Light-Emitting Diode, OLED), or the like. The touch panel 111, also referred to as a touchscreen, a touch-sensitive screen, or the like, may collect a touch or non-touch operation (for example, an operation performed by the user on the touch panel 111 or near the touch panel 111 by using any proper object or accessory, such as a finger or a stylus, or a motion sensing operation may be included, and the operation includes an operation type such as a single-point control operation or a multipoint control operation) performed by the user on or near the touch panel 111, and drive a corresponding connection apparatus based on a preset program. It should be noted that the touch panel 111 may further include two parts: a touch detection apparatus and a touch controller. The touch detection apparatus detects a touch orientation and gesture of the user, detects a signal brought by the touch operation, and sends the signal to the touch controller. The touch controller receives touch information from the touch detection apparatus, converts the touch information into information that can be processed by the processor 101, and then sends the information to the processor 101. In addition, the touch controller can further receive and execute a command sent by the processor 101. In addition, the touch panel 111 may be implemented in a plurality of types, such as a resistive type, a capacitive type, an infrared ray, and a surface acoustic wave, or the touch panel 111 may be implemented by using any technology developed in the future. Generally, the touch panel 111 may cover the display panel 112. The user may perform, based on content displayed on the display panel 112 (the displayed content includes but is not limited to a soft keyboard, a virtual mouse, a virtual key, an icon, and the like), an operation on or near the touch panel 111 that covers the display panel 112. After detecting the operation on or near the touch panel 111, the touch panel 111 sends the operation to the processor 101 to determine the user input, and then the processor 101 provides a corresponding visual output on the display panel 112 based on the user input. In FIG. 1, the touch panel 111 and the display panel 112 serve as two independent components to implement input and output functions of the terminal 100. However, in some embodiments, the touch panel 111 and the display panel 112 may be integrated to implement the input and output functions of the terminal 100.

The RF circuit 104, the loudspeaker 106, and the microphone 107 may provide an audio interface between the user and the terminal 100. The audio circuit 105 may convert received audio data into a signal, and transmit the signal to the loudspeaker 106, and the loudspeaker 106 converts the signal into a voice signal for output. In addition, the microphone 107 may convert a collected voice signal into a signal, and the audio circuit 105 receives the signal, converts the signal into audio data, and then outputs the audio data to the RF circuit 104, to send the audio data to a device such as another terminal, or output the audio data to the memory 102, so that the processor 101 performs further processing with reference to content stored in the memory 102. In addition, the camera 103 may collect an image frame in real time and send the image frame to the processor 101 for further processing, and store a processed result in the memory 102 and/or display the processed result to the user by using the display panel 112.

The processor 101 is a control center of the terminal 100, is connected to each part of the entire terminal 100 by using various interfaces and lines, and performs various functions of the terminal 100 and data processing by running or executing the software program and/or a module stored in the memory 102 and invoking data stored in the memory 102, to perform overall monitoring on the terminal 100. It should be noted that the processor 101 may include one or more processing units. The processor 101 may further integrate an application processor and a modem processor, or may be an application processor or a modem. The application processor mainly processes an operating system, a user interface (User Interface, UI), an application program, and the like, and the modem processor mainly processes wireless communication.

The terminal 100 may further include the power supply 114 (for example, a battery) that supplies power to each component. In the embodiments of the present invention, the power supply 114 may be connected to the processor 101 by using a power management system, so as to implement functions such as charging management, discharging management, and power consumption management by using the power management system.

In addition, a component not shown in FIG. 1 also exists. For example, the terminal 100 may further include a Bluetooth module, and the like. Details are not described herein.

An embodiment of the present invention provides a stress detection method. The method may be performed by the terminal 100, or certainly may be performed by at least two devices in cooperation, for example, a wearable device and the terminal 100 in cooperation.

The following describes the method provided in this embodiment of the present invention by using a single terminal as an example. As shown in FIG. 2, the method includes S301 to S303.

S301. Obtain an event of the terminal.

The event may be at least one of a schedule, a reminder, or a running application program.

In this embodiment of the present invention, the schedule and the reminder can reflect at least an event type and an even occurrence time. For example, the schedule and the reminder include but are not limited to an application program that has a timing reminder function, such as a calendar or an alarm. In the application program, information such as a location, a time, and content of an event may be selectively recorded. This is not limited herein.

It may be understood that the terminal may also extract an event and an event occurrence time based on received information (for example, an SMS message). This manner may also be understood as a reminder.

The terminal may determine the running application program in one of the following implementations.

For an application program running in the foreground, the terminal may obtain the application program based on a name of the application program. The application program running in the foreground may be a currently used program. To be specific, the application program directly runs by using a window, and can interact with a user by using a display screen.

When the terminal receives an operation that the user taps an application icon, the terminal presents, to the user, a home screen of an application program corresponding to the application icon. Alternatively, if an application program is running in the background, the terminal presents, to the user, a screen that is finally presented before the application program is switched to the background. In this case, the terminal may use the application program as a running application program. Running in the background may mean that an application program is not used and is not disabled after being enabled. An application program running in the background is usually invisible, that is, the application program runs in a resource manager and usually no screen appears. However, running of the application program occupies system resources, and the application program may provide an auxiliary function for another application program.

When a new application program is enabled, the application program reports an event to a system of the terminal. After the event is reported, the system adds the application program to a list of applications running in the system. In this case, the terminal may also identify the application program corresponding to content added to the application list as a newly enabled application program.

The foregoing case is a possible implementation in which the terminal identifies the currently running application program, but is not intended to limit this embodiment of the present invention.

S302. Determine a stress measurement time based on the event of the terminal.

Considering that different events correspond to different stress measurement times, in this embodiment of the present invention, after a type of an event is determined, stress is measured at different times for different types of events. A specific manner of determining the stress measurement time is described in detail below, and details are not described herein. It should be noted that the determined stress measurement time may be a time point or a time period. When the stress measurement time is a time period, the stress is measured in the time period.

S303. Detect stress of the user based on the time.

After the stress measurement time point or time period corresponding to the event of the terminal is determined, stress measurement is performed at the time point, or stress measurement is performed in real time in the time period. Certainly, to reduce power consumption caused by the stress measurement, stress measurement may be performed at a preset time point in the time period.

It can be learned that, the stress measurement time is determined based on the obtained event of the terminal, and the stress of the user is measured based on the time, so that the stress measurement time can be more targeted, to improve reliability of data obtained through stress measurement. In addition, real-time stress measurement further reduces power consumption of the terminal.

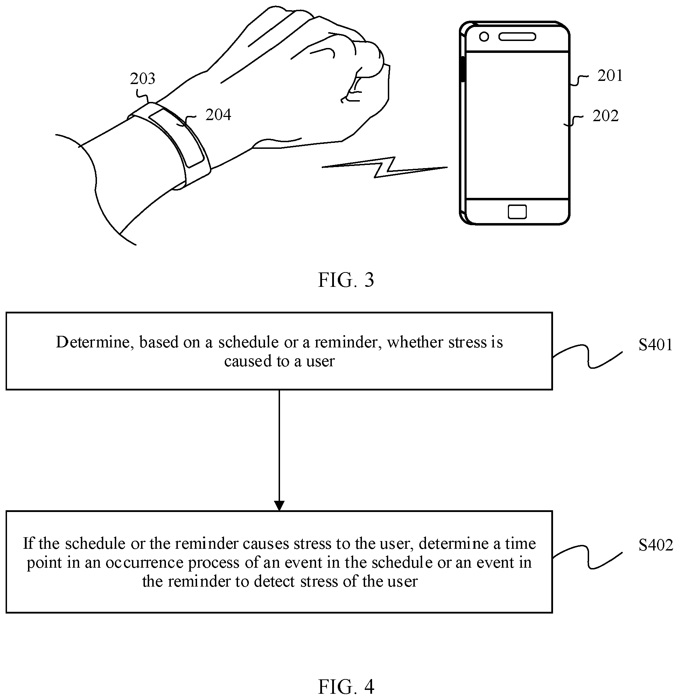

It should be noted that an application scenario of this embodiment of the present invention is that at least two devices cooperate with each other. For example, a band 203 and a mobile phone 201 shown in FIG. 3 cooperate with each other. In this case, the mobile phone may be configured to obtain an event of the mobile phone, and determine a stress measurement time based on the event. Then, the mobile phone may transmit the stress measurement time to the band in a transmission manner such as a network or Bluetooth, so that the band measures the stress of the user based on the time. It should be noted that, because the mobile phone has a stronger computing capability, and more power compared with the band, the band only needs to perform stress measurement based on the time determined by the mobile phone, and may not analyze the event. Certainly, if the user currently requires the band to implement more functions implemented by the mobile phone, after the mobile phone obtains the event, the band may obtain the event from the mobile phone, determine the stress measurement time based on the event, and measure the stress of the user based on the time.

It can be learned that, in this embodiment of the present invention, in the scenario in which at least two devices cooperate with each other, a connection manner between devices in the at least two devices, a function of each device, and the like are not limited. Certainly, the cooperation between the at least two devices are not limited to cooperation between the mobile phone and the band, and may be cooperation between a mobile terminal and a wearable device, cooperation between at least two mobile terminals, cooperation between at least two wearable devices, or the like. This is not limited herein. The wearable device includes but is not limited to a device such as a band, a watch, or a Bluetooth headset. The mobile terminal includes but is not limited to a device such as a mobile phone or a tablet computer.

If the event is at least one of the schedule and the reminder, the determining a stress measurement time based on the event of the terminal, and detecting stress of the user based on the time in S302 and S303 may be specifically implemented as S401 and S402 shown in FIG. 4.

S401. Determine, based on the schedule or the reminder, whether stress is caused to the user.

S402. If the schedule or the reminder causes stress to the user, determine a time point in an occurrence process of an event in the schedule or an event in the reminder to detect the stress of the user.

Content in S401 and S402 is described below with reference to a specific example.

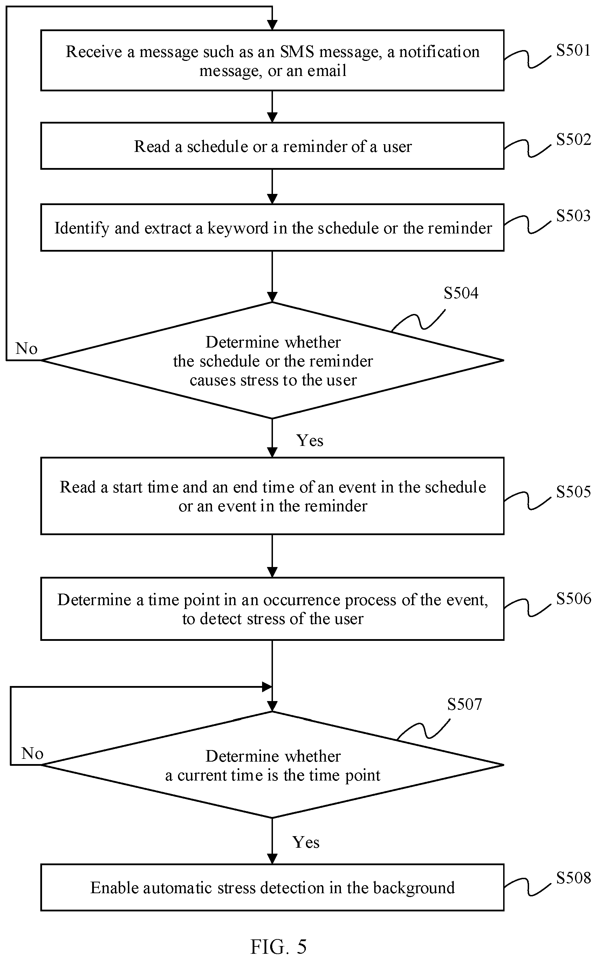

FIG. 5 is a flowchart of a method procedure in which a mobile phone identifies an event in a schedule or an event in a reminder, determines a stress detection time point, and subsequently implements stress detection according to an embodiment of the present invention. The method procedure includes S501 to S508.

S501. Receive a message such as an SMS message, a notification message, or an email.

S502. Read a schedule or a reminder of a user.

S503. Identify and extract a keyword in the schedule or the reminder.

S504. Determine whether the schedule or the reminder causes stress to the user. If yes, S505 is performed; otherwise, S501 is performed again. After a new message such as a short message, a notification message, or an email is received, this cyclic process is triggered.

Considering that different users have different reactions to different types of events, in this embodiment of the present invention, the user may preset, based on a situation of the user, events that cause stress to the user, in other words, events that cause a change to the stress of the user. The mobile phone may locally store content that is set by the user, or store the content in a third-party device such as a server, or store the content in a cloud, and obtain the content from a storage location when the content is needed. In addition, the mobile phone may also determine, through deep learning, events that are more likely to cause stress to the user, and then select, through filtering, events that may cause stress to the user.

For example, if the user receives an SMS message: "See you in the western restaurant at 6:00 p.m. on November 2", the mobile phone may obtain keywords such as "November 2", "6:00 p.m.", and "west restaurant". Then, the mobile phone uses 6:00 p.m. on November 2 as an occurrence time of the event, and determines, based on the western restaurant, that an event type is an appointment. Generally, an appointment may make the user feel happy. Therefore, it is determined that the event type can affect the stress of the user, and the event type meets a condition for stress detection.

In this embodiment of the present invention, the mobile phone may further selectively use, as a to-be-detected event based on an operation of the user, an event involved in a received message such as an SMS message, a notification message, or an email that partially carries a keyword.

For example, after the user receives an SMS message: "See you in the western restaurant at 6:00 p.m. on November 2", and replies with an SMS message including keywords such as "Ok", "All right", and "No problem", it is determined that the event identified by the mobile phone is a to-be-detected event. Because the user replies with a positive answer, it may be determined that such an event usually occurs on schedule. In this way, reliability of a detection process is further improved. However, when only the SMS message is received but no reply content of the user is detected in a specific time, the user may be asked, at an appropriate time in a manner such as a pop-up dialog box, whether the event is to occur, so that after it is determined that the event is to occur, a stress measurement time corresponding to the event is determined.

In addition, the mobile phone may further determine, based on an operation performed by the user on the received message, whether detection is required in an occurrence process of the event.

For example, after receiving an SMS message: "See you in the western restaurant at 6:00 p.m. on November 2", the user manually adds the SMS message to an option that has a prompt function, such as the schedule or the reminder. In this case, the mobile phone may determine that the user is likely to attend the appointment at 6:00 p.m. on November 2. A process in which the user manually adds the SMS message to the option that has the prompt function, such as the schedule or the reminder may be embodied as adding a label to the SMS message, sticking the SMS message on top, or directly importing content of the SMS message into an application that has a prompt function, such as a calendar. This is not limited herein.

It can be learned that the mobile phone determines, in a plurality of manners, whether an event causes stress to the user. For example, a currently obtained event of the mobile phone may be compared with an event that is preset by the user and that may cause stress to the user, to determine whether the event causes stress to the user. Certainly, a type of a current event may also be determined, to determine, with reference to a historical empirical value, whether the type of event causes stress to the user.

In addition, the mobile phone may further directly select, from a schedule or a reminder that has been imported or established in the mobile phone, an event that causes stress to the user. To be specific, the mobile phone may directly access an option that has a prompt function, such as the schedule or the reminder, and obtain a type of each event from the option, and after it is determined that the type of event is a type that may cause a change to the stress of the user, such as an appointment, an exam, a meeting, or travel arrangement, the event is used as a to-be-detected event.

S505. Read a start time and an end time of the event in the schedule or the event in the reminder.

The mobile phone may read the start time and the end time of the event from the schedule or the reminder. It may be understood that alternatively, the mobile phone may identify the start time of the event, for example, 6:00 p.m. on November 2 in the foregoing example, but cannot determine the end time of the event. The mobile phone can calculate, based on a historical empirical value or user-preset duration that may be occupied by an appointment event, for example, two hours, that the event is to occur from 6:00 to 8:00 p.m. on November 2. In this case, the mobile phone may use 6:00 to 8:00 p.m. on November 2 as a time period in which the event occurs. The mobile phone may alternatively identify the end time of the event but cannot identify the start time of the event. In this case, the mobile phone may calculate the start time of the event in a manner similar to that in the foregoing example, to determine the start time and the end time of the event.

S506. Determine a time point in an occurrence process of the event, to detect the stress of the user.

The occurrence process of the event refers to a time period formed by using the start time of the event as a start moment and using the end time of the event as an end moment.

When the start time of the event is known but the end time of the event is not determined, considering that the end time of the event is calculated by the mobile phone and is less accurate than the start time of the event, the mobile phone may directly detect the stress at the start time, or select the start time as a reference, and detect the stress at a cut-off moment of a time period after the start time, to improve reliability of detected stress. The time period may be preset by the user, and for example, may be set to 2 minutes.

Similarly, when the end time of the event is known but the start time of the event is not determined, considering that the start time of the event is calculated by the mobile phone and is far less accurate than the end time of the event, the mobile phone may directly detect the stress at the end time, or select the end time as a reference, and detect the stress at a start moment of a time period before the end time, to improve reliability of detected stress. The time period may alternatively be preset by the user, and for example, may be set to 3 minutes.

When the start time and the end time of the event are known, the mobile phone may detect the stress of the user at the start time, at the end time, at a time point or in a time period in the occurrence process, or the like. It may be understood that, when the start time of the event is known, the mobile phone may also detect the stress of the user at a time point or in a time period after the start time of the event. When the end time of the event is known, the mobile phone may also detect the stress of the user at a time point or in a time period before the end time of the event.

It should be noted that the stress detection time points may be preset by the user, and a setting manner includes but is not limited to the foregoing examples. Details are not described herein again.

In addition, in this embodiment of the present invention, a quantity of stress detection time points is not limited. If more energy consumption needs to be reduced, fewer stress detection time points are set. If collection precision needs to be ensured, more stress detection time points are set. Alternatively, energy consumption and precision may be balanced, and a relatively proper quantity of time points are set as the stress detection time points.

S507. Determine whether a current time is the time point. If yes, S508 is performed; otherwise, S507 is performed again, and whether the time point arrives continues to be determined.

S508. Enable automatic stress detection in the background.

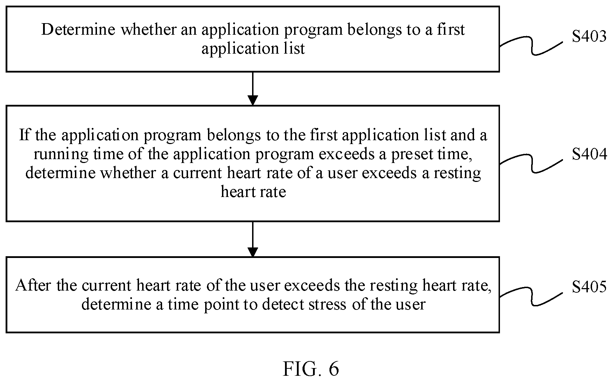

If the event is the running application program, the determining a stress measurement time based on the event of the terminal, and detecting stress of the user based on the time in S302 and S303 may be implemented as S403 to S405 in FIG. 6.

S403. Determine whether the application program belongs to a first application list.

The first application list is a list of application programs that affect the stress of the user.

In this embodiment of the present invention, an application program recorded in the first application list is an application program that affects the stress of the user. That is, during running of the application program, the stress of the user distinctly changes. The user may preset the first application list. Alternatively, the user sets a type of application program that affects the stress of the user, and then the mobile phone may automatically select application programs of the type, and use the selected application programs as the first application list. It should be noted that a manner of setting the first application list is not limited herein.

S404. If the application program belongs to the first application list and a running time of the application program exceeds a preset time, determine whether a current heart rate of the user exceeds a resting heart rate.

It should be noted that different application programs bring different impacts to the stress of the user during running, and start times of the impacts are not necessarily the same. Therefore, in this embodiment of the present invention, a preset time is usually preset for each application program. Certainly, a same preset time or different preset times may be set for different application programs based on characteristics of the application programs. For example, the preset time is set based on a type of the application program, or the preset time is set based on a function of the application program. A manner of setting the preset time is not limited herein. The preset time indicates that after the user accesses the application program, when the preset time is reached, the stress of the user changes.

S405. After the current heart rate of the user exceeds the resting heart rate, determine a time point to detect the stress of the user.

To ensure reliability of measured stress, the stress of the user may be detected after a time period after it is determined that the running time of the application program exceeds the preset time. In this case, the heart rate of the user already exceeds the resting heart rate, which means that a currently running application program has affected the stress of the user, and the affect lasts for a time period. In this way, the detected stress is more reliable.

The resting heart rate refers to a quantity of heartbeats per minute when the user is in a waking, inactive, and quiet state. It may be understood that, similar to the resting heart rate, names such as a quiet heart rate and a steady heart rate may be understood as the resting heart rate.

It should be noted that the determined stress detection time point may be preset by the user according to factors such as a bearing capability of the user. For example, for a user whose emotion is prone to fluctuate, the time point may be set to a time point relatively close to a time point at which a current heart rate of the user exceeds the resting heart rate. For a user with a relatively good emotion control capability, the time point may be set to a time point relatively far from a time point at which a current heart rate of the user exceeds the resting heart rate. In this way, a time point suitable for current user stress detection is set based on bearing capabilities of different users.

In this embodiment of the present invention, a setting manner, a value, and the like of the time point are not limited.

Content in S403 to S405 is described below with reference to a specific example.

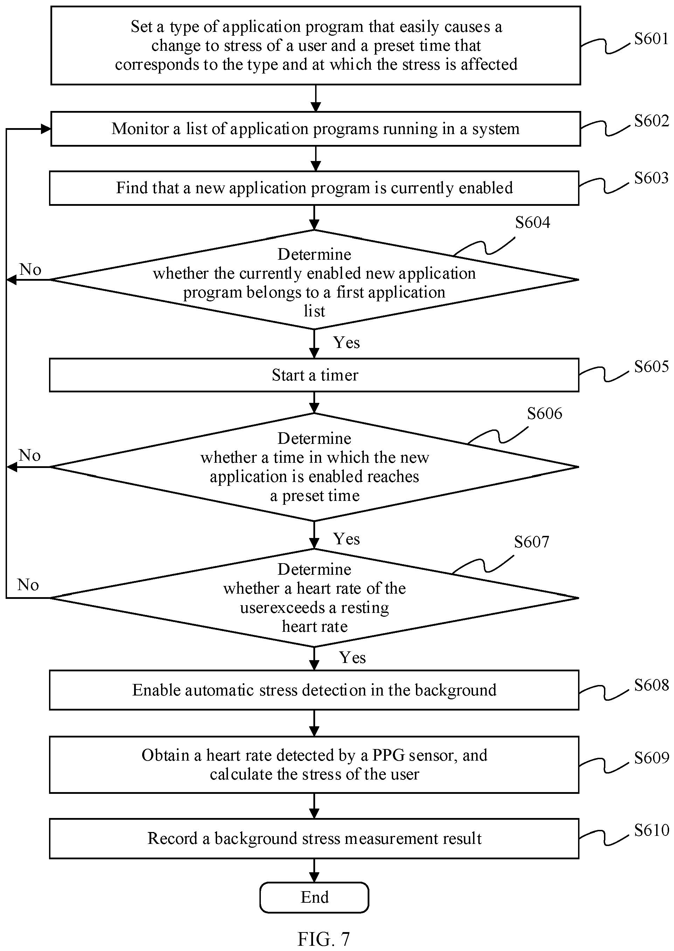

FIG. 7 is a flowchart of a method procedure in which a mobile phone identifies a running application program, determines a stress detection time point if the application program belongs to a first application list, and subsequently implements stress detection according to an embodiment of the present invention. The method procedure includes S601 to S610.

S601. Set a type of application program that easily causes a change to stress of a user and a preset time that corresponds to the type and at which the stress is affected.

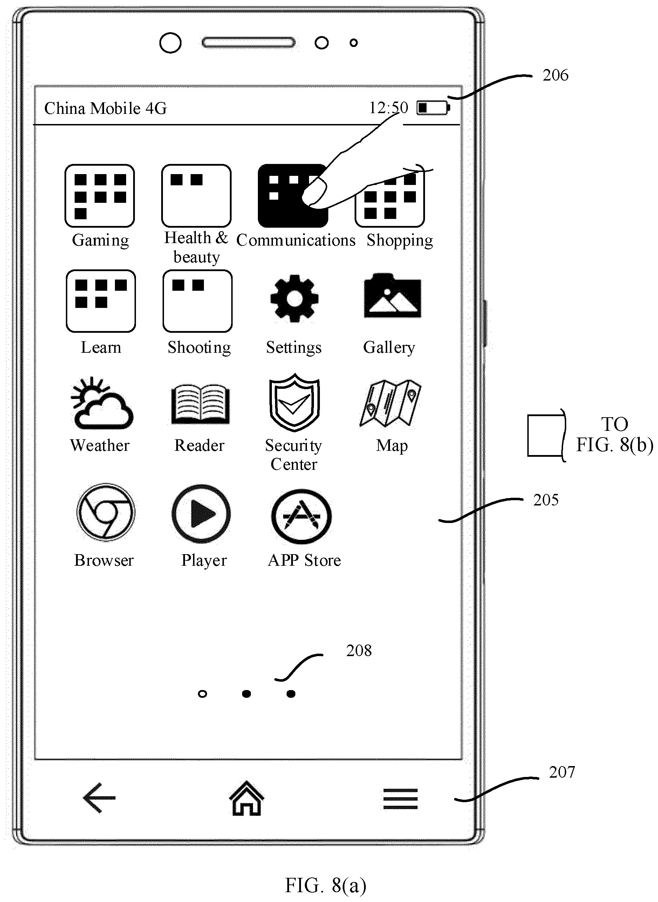

In this embodiment of the present invention, the user may classify, in advance, application programs installed on the mobile phone. For example, FIG. 8(a) shows a display screen obtained after the user classifies the application programs on the mobile phone.

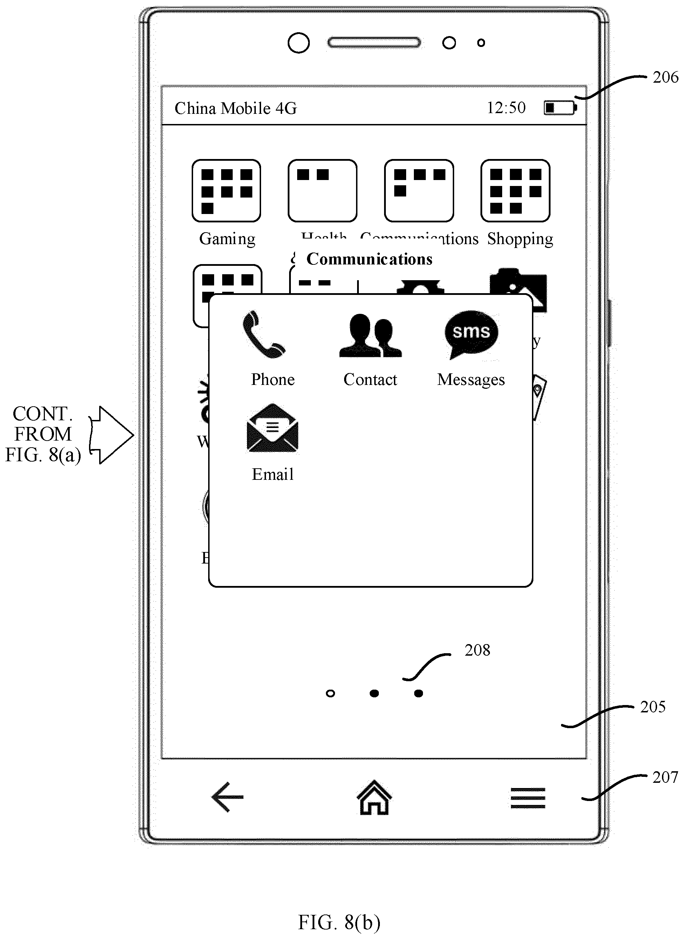

In the screen shown in FIG. 8(a), each folder may be considered as a classification created by the user, and a name of the folder may be set by the user before the classification, during the classification, or after the classification. A folder named "Communication" is used as an example. After the user taps the folder, a display screen shown in FIG. 8(b) is displayed on the mobile phone. The folder includes application programs named Phone, Contacts, Messages, and Email. These application programs may be considered as application programs of a communication type, and the user may classify the application programs into this type based on a definition of a function of each application program.

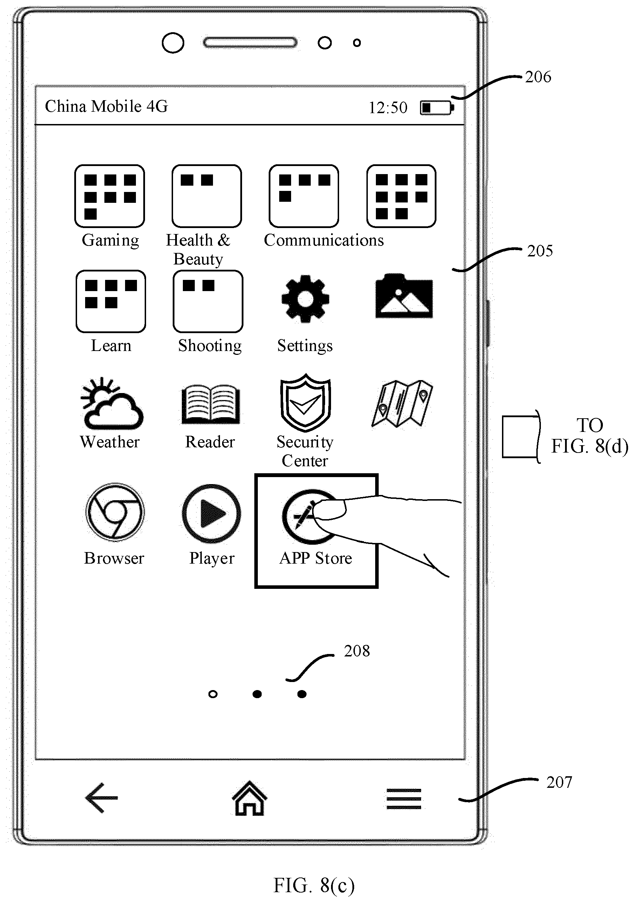

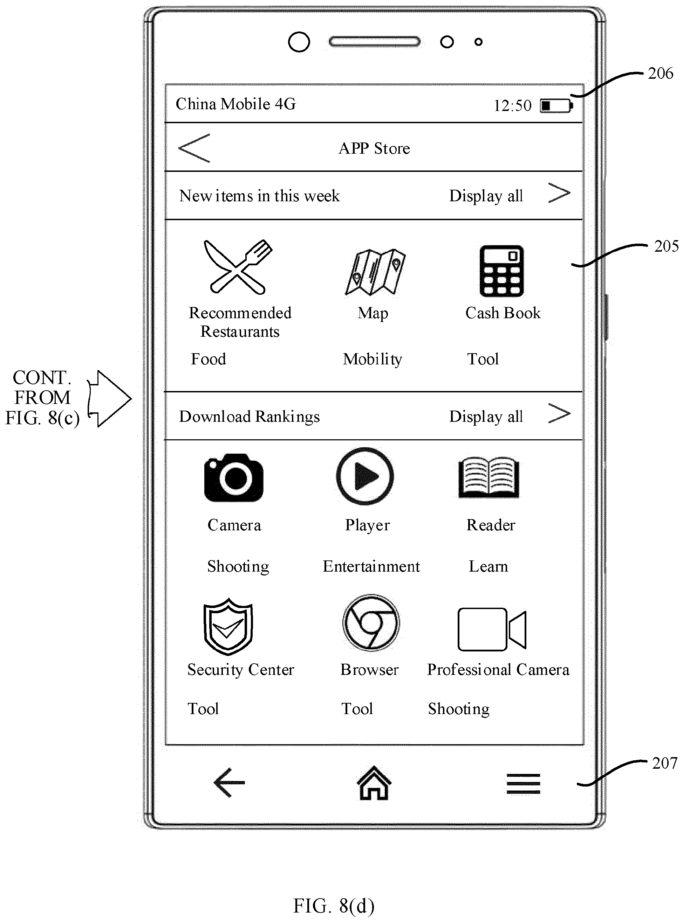

For another example, as shown in FIG. 8(c), the user may access, through tapping, a platform such as an application store that is provided for the mobile phone to update and download an application program. In the application store, as shown in FIG. 8(d), when an icon and a name of each application program are presented, a type of the application program may be presented to the user. The type is usually a category into which the application store classifies the application. During classification, the application programs may be classified into categories, in other words, types of the application programs based on functions of the application programs, or the like. In the application store, content such as a text or an icon that is used to indicate a type of an application program may be intuitively presented to the user or hidden. When the content is hidden, when the user requires the content, the content may be triggered in a manner such as tapping or sliding performed by the user on a screen of the mobile phone, so that the content is displayed.

It can be learned that when determining a type to which an application program currently accessed by the user belongs, the mobile phone may determine, with reference to classification of types of the application program in the application store, whether the application program belongs to the first application list.

In this embodiment of the present invention, a type of an application program that may cause a change to the stress of the user may be automatically set by the mobile phone, or may be manually set by the user. This is not limited herein.

It should be noted that different types of application programs may bring different experience to the user. Therefore, in this embodiment of the present invention, preset times that are corresponding to different types of application programs and at which the stress is affected are usually different. For example, for a game application program, the user usually gets nervous soon after accessing the application program. Then, a preset time of the game application program may be set to 1 minute or even shorter. For another example, for a learning application program, the user usually generates emotions such as fatigue and resistance after using the application program for a relatively long time. Then, a preset time of the learning application program may be set to 30 minutes or even longer. Certainly, for ease of setting, the preset times may also be set to a same parameter.

S602. Monitor a list of application programs running in a system.

S603. Find that a new application program is currently enabled.

S604. Determine whether the new application program that is currently enabled belongs to the first application list. If yes, S605 is performed; otherwise, the list of applications running in the system continues to be monitored.

S605. Start a timer.

In this embodiment of the present invention, time of the timer may be preset by the user based on a measurement requirement of the user. Certainly, the time of the timer may alternatively be preset before the mobile phone is delivered from a factory. The timer may record, in a countdown manner, a time in which an application program runs. For example, the timer may use the preset time set in S601 as a start point to start countdown.

S606. Determine whether a time in which the new application program is enabled reaches a preset time. If yes, S607 is performed; otherwise, the list of applications running in the system continues to be monitored.

To be specific, after the new application is enabled, if the new application still runs when the timer counts down to 0, S607 may be performed.

S607. Determine whether a heart rate of the user exceeds a resting heart rate. If yes, S608 is performed; otherwise, S602 is performed.

S608. Enable automatic stress detection in the background.

Considering that when emotion of the user fluctuates greatly, the detected heart rate usually exceeds the resting heart rate, in this embodiment of the present invention, after the heart rate of the user exceeds the resting heart rate, the automatic stress detection in the background is enabled. In this way, energy consumption for real-time stress detection can be reduced while relatively high reliability of stress measurement is ensured.

S609. Obtain a heart rate detected by a PPG sensor, and calculate the stress of the user.

A manner of calculating the stress of the user is proposed in the following, and details are not described herein.

S610. Record a background stress measurement result.

It can be learned that, considering that the stress of the user usually do not change when the started new application program does not belong to the first application list, when the running time of the started new application program does not reach the preset time, and when the heart rate of the user does not exceed the resting heart rate, to further save power, resources, and the like consumed for stress detection, the stress of the user may not be detected in the foregoing cases in this embodiment of the present invention.

It may be understood that the method procedure from S601 to S610 is described as an embodiment. S602 may alternatively be omitted in the method procedure. To be specific, the list of the application programs running in the system is not monitored, and S603 may be performed. In this case, when it is determined in S604 that the currently started new application program does not belong to the first application list (that is, a branch in which a determining result is no in S604), S603 may be performed. When it is determined in S606 that a time in which the new application program is enabled does not reach the preset time (that is, a branch in which a determining result is no in S606), S603 may be performed. When it is determined in S607 that the heart rate of the user does not exceed the resting heart rate (that is, a branch in which a determining result is no in S607), S603 may be performed.

Another stress detection method provided in this embodiment of the present invention may also be performed by the terminal 100, or certainly may be performed by at least two devices in cooperation. At least one device in the at least two devices is implemented as the terminal 100.

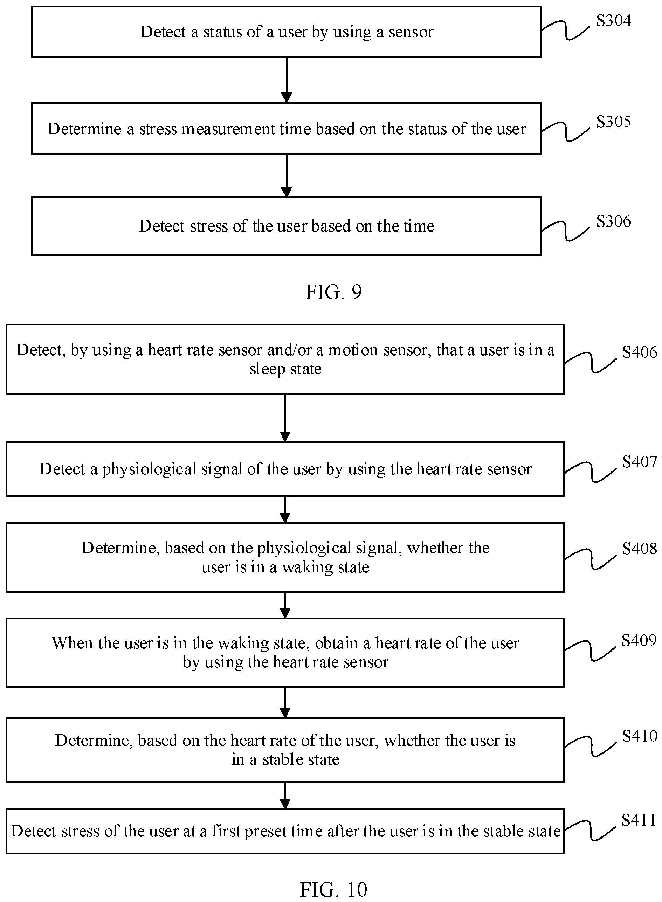

The following describes the method provided in this embodiment of the present invention still by using a single terminal as an example. As shown in FIG. 9, the method includes S304 to S306.

S304. Detect a status of a user by using a sensor.

A wearable device is used as an example. The wearable device may detect the status of the user by using a heart rate sensor, a motion sensor, or a heart rate sensor and a motion sensor. The wearable device may detect a heart rate of the user by using the heart rate sensor, and then monitor a change of the heart rate in a time period, to determine the status of the user. The change of the heart rate includes but is not limited to a change amplitude of intervals between adjacent peaks, peak values, adjacent periods, and the like in a heart rate curve. The wearable device may further detect an acceleration of the user by using the motion sensor, and then monitor a change of the acceleration in a time period, to determine the status of the user. The change of the acceleration includes but is not limited to a change of an acceleration value, a change of an acceleration direction, a change amplitude of the acceleration, and the like. Similarly, the wearable device may also determine the status of the user with reference to monitoring statuses of both the heart rate sensor and the motion sensor in a time period, so that the determined status of the user can more intuitively reflect an actual status of the user. The heart rate sensor includes but is not limited to a PPG, and the motion sensor includes but is not limited to an ACC.

In this embodiment of the present invention, the status of the user includes but is not limited to a sleep state, a waking state, a stable state, a moving state, and the like. Information such as a definition and a feature of each state is provided in the following, and details are not described herein. It should be noted that, when the user is in different states, there may be more or less differences between information detected by using the sensor or between signals detected by using the sensor, provided that the status of the user can be identified by using the wearable device.

S305. Determine a stress measurement time based on the status of the user.

Considering that different states correspond to different stress measurement times, in this embodiment of the present invention, after the status of the user is determined, stress is measured at different times for different states. A specific manner of determining the stress measurement time is described in detail below, and details are not described herein. It should be noted that the determined stress measurement time may be a time point or a time period, provided that the stress is measured within the time period.

S306. Detect stress of the user based on the time.

After the stress measurement time point or time period corresponding to the status of the user is determined, stress measurement is performed at the time point, or stress measurement is performed in real time in the time period. Certainly, to reduce energy consumption caused by the stress measurement, stress measurement may alternatively be performed at a preset time point in the time period.

It can be learned that, the stress measurement time is determined based on the obtained status of the user, and the stress of the user is detected based on the time, so that the stress measurement time can be more targeted, to improve reliability of data obtained through stress measurement.

It should be noted that an application scenario of this embodiment of the present invention is that at least two devices cooperate with each other, for example, a band and a mobile phone shown in FIG. 3 cooperate with each other. In this case, the band may be configured to obtain a parameter used to determine the status of the user. Then, the band may transmit the parameter to the mobile phone in a transmission manner such as a network, Bluetooth, or Wi-Fi, and the mobile phone determines the status of the user. The mobile phone may determine the stress measurement time based on the status of the user, and then the mobile phone transmits the stress measurement time to the band, so that the band detects the stress of the user based on the time. It should be noted that, because the mobile phone has a stronger computing capability, and more power compared with the band, the band only needs to collect the parameter when the status of the user is determined, and perform stress measurement based on the time determined by the mobile phone, and may not analyze the status of the user. Certainly, if the user currently requires the band to implement more functions implemented by the mobile phone, the band may determine the status of the user based on the collected parameter, determine the stress measurement time based on the status, and measure the stress of the user based on the time.

It can be learned that, in this embodiment of the present invention, in a scenario in which at least two devices cooperate with each other, a connection manner between devices in the at least two devices, a function of each device, and the like are not limited. Certainly, the cooperation between the at least two devices are not limited to cooperation between the mobile phone and the band, and may be cooperation between a mobile terminal and a wearable device, cooperation between at least two mobile terminals, cooperation between at least two wearable devices, or the like. This is not limited herein. The wearable device includes but is not limited to a device such as a band, a watch, or a Bluetooth headset. The mobile terminal includes but is not limited to a device such as a mobile phone or a tablet computer.

For example, as shown in FIG. 10, the detecting a status of a user by using a sensor in S304 may be specifically implemented as S406 to S408. The determining a stress measurement time based on the status of the user, and detecting stress of the user based on the time in S305 and S306 may be specifically implemented as S409 to S411.

S406. Detect, by using a heart rate sensor and/or a motion sensor, that the user is in a sleep state.

The wearable device may detect, by using a PPG sensor, a heart rate signal used to indicate a heart rate of the user, and determine a current heart rate of the user based on the heart rate signal. If a value of the heart rate falls within a heart rate threshold range in the sleep state, it is determined that the user is currently in the sleep state. If a heart rate of the user detected at a next time point does not belong to the heart rate threshold range in the sleep state, or belongs to a heart rate threshold range in a waking state, it is determined that the user is currently in the waking state.

A heart rate threshold range in each state may be preset based on an empirical value, or may be preset by the user based on a physical condition of the user. A manner of setting the heart rate threshold range is not limited in this embodiment of the present invention.

The wearable device may further detect an acceleration of the user by using an ACC sensor. If the detected acceleration is greater than an acceleration threshold, it is determined that the user is in the waking state; otherwise, if the detected acceleration is less than or equal to the acceleration threshold, it is determined that the user is in the sleep state. For example, if the acceleration threshold is 0.5g (a value of g is usually 9.8 m/s²), an acceleration of the user in the sleep state is less than or equal to 0.5g. If it is detected that an acceleration of the user is 2g, because 2g is greater than the acceleration threshold, it is determined that the user is in the waking state.

The wearable device may further detect duration of continuous movement of the user by using the ACC sensor, and then compare the detected duration of continuous movement with a duration threshold of continuous movement of the user in the sleep state. If the detected duration is less than the duration threshold, the user is in the sleep state; otherwise, the user is in the waking state. For example, if the wearable device detects that the user keeps moving for one minute, and one minute exceeds the duration threshold, the wearable device determines that the user is in the waking state.

It should be noted that the foregoing manner of determining whether the user is in the sleep state or the waking state is a possible implementation. In this embodiment of the present invention, a manner of determining the status of the user is not limited.

S407. Detect a physiological signal of the user by using the heart rate sensor.

In this embodiment of the present invention, the physiological signal includes the heart rate of the user.

S408. Determine, based on the physiological signal, whether the user is in the waking state.

Considering that when the user is in the waking state, the user probably does not do strenuous exercise in a time period, and the acceleration of the user may not be detected by using the ACC sensor, the heart rate can more effectively reflect that the user enters the waking state from the sleep state.

The waking state is a state in which the user wakes up from the sleep state.

S409. When the user is in the waking state, obtain a heart rate of the user by using the heart rate sensor.

S410. Determine, based on the heart rate of the user, whether the user is in a stable state.

In this embodiment of the present invention, the stable state may be understood as a state in which the user does a low-intensity activity, in other words, the current heart rate of the user tends to be stable. In this case, detected stress can better reflect an actual status of the user. The low-intensity activity includes but is not limited to a small-scale activity such as walking or reading a book that almost does not cause a change to the heart rate of the user.

S411. Detect stress of the user at a first preset time after the user is in the stable state.

The first preset time may be preset by the user based on a historical empirical value. Considering that the wearable device may incorrectly determine the state when the user just enters the stable state, when the stable state lasts for/until the first preset time, it may be considered that the user currently is truly in the stable state. In this case, the detected stress can more effectively reflect the actual status of the user.

Content in S406 to S411 is described below with reference to a specific example.

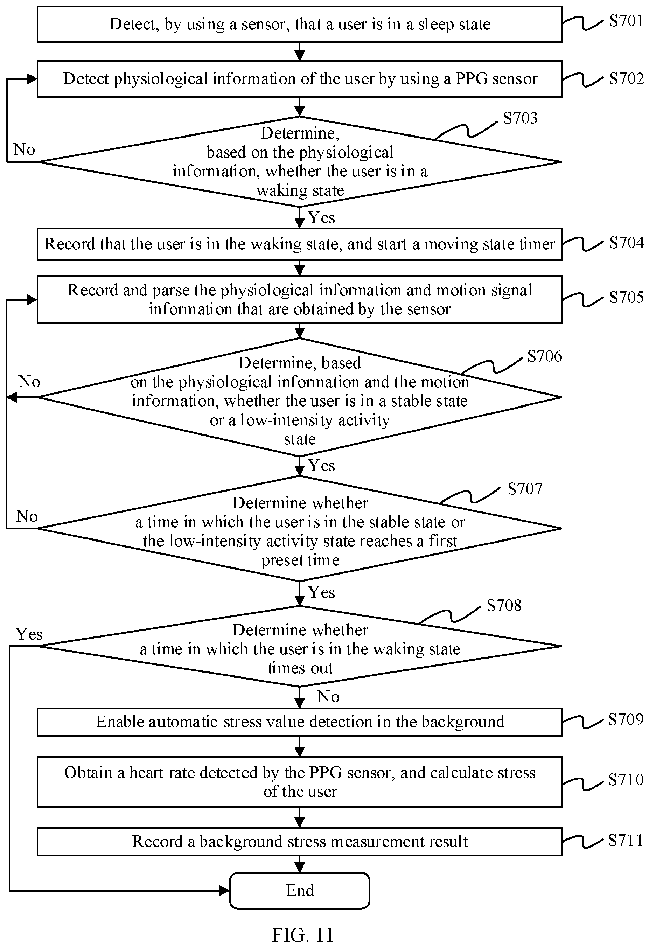

FIG. 11 shows a method procedure in which a wearable device identifies a waking state of a user by using a sensor, and detects stress of the user when the user is in a stable state according to an embodiment of the present invention. The method procedure includes S701 to S711.

S701. Detect, by using the sensor, that the user is in a sleep state.

For a process of determining whether the user is in the sleep state, refer to the foregoing description. Details are not described herein again.

S702. Detect physiological information of the user by using a PPG sensor.

S703. Determine, based on the physiological information, whether the user is in the waking state. If yes, S704 is performed; otherwise, the physiological information of the user continues to be detected.

S704. Record that the user is in the waking state, and start a moving state timer.

In this embodiment of the present invention, time of the moving state timer may be preset by the user based on a measurement requirement of the user. The moving state timer may record, through countdown, a time in which the user is in the waking state.

S705. Record and parse the physiological information and motion signal information that are obtained by the sensor.

In this embodiment of the present invention, the motion signal information is mainly from an ACC sensor. To be specific, the motion information includes an acceleration of the user. The physiological information is mainly from a PPG sensor. To be specific, the physiological information includes a heart rate of the user.

S706. Determine, based on the physiological information and the motion information, whether the user is in a stable state or a low-intensity activity state. If yes, S707 is performed; otherwise, the information collected by the sensor continues to be recorded and parsed.

For an implementation of determining whether the user is in the stable state or the low-intensity activity state, refer to the foregoing description. Details are not described herein again.

S707. Determine whether a time in which the user is in the stable state or the low-intensity activity state reaches a first preset time. If yes, S708 is performed; otherwise, the information collected by the sensor continues to be recorded and parsed.

After the time in which the user is in the stable state or the low-intensity activity state reaches the first preset time, it may be considered that stress of the user detected in this case is relatively accurate.

S708. Determine whether the time in which the user is in the waking state expires. If yes, S709 is performed; otherwise, this stress value detection procedure ends.

In this case, for determining whether the time in which the user is in the waking state expires, refer to description of the moving state timer enabled in S704. If the moving state timer has not counted down to 0 currently, it is considered that the time in which the user is in the waking state has not expired. Otherwise, it is considered that the time in which the user is in the waking state expires.

S709. Enable automatic stress detection in the background.

S710. Obtain a heart rate detected by the PPG sensor, and calculate the stress of the user.

A manner of calculating the stress of the user may be a manner of calculating the stress of the user with reference to the heart rate in the prior art, and details are not described herein.

S711. Record a background stress measurement result.

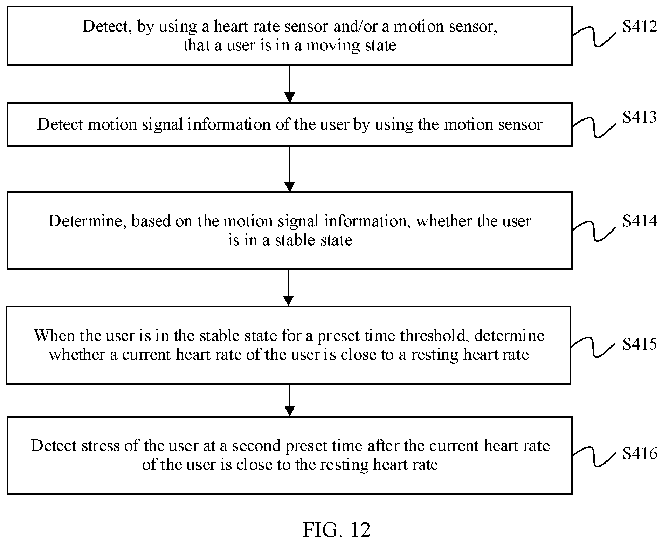

For example, as shown in FIG. 12, the detecting a status of a user by using a sensor in S304 may be specifically implemented as S412 to S414. The determining a stress measurement time based on the status of the user, and detecting stress of the user based on the time in S305 and S306 may be specifically implemented as S415 and S416.

S412. Detect, by using a heart rate sensor and/or a motion sensor, that the user is in a moving state.

The moving state includes but is not limited to a high-intensity moving state such as running or heel-and-toe walking. Compared with a stable state, a heart rate of the user in the moving state usually fluctuates more greatly.

S413. Detect motion signal information of the user by using the motion sensor.

S414. Determine, based on the motion signal information, whether the user is in the stable state.

S415. When the user is in the stable state for a preset time threshold, determine whether a current heart rate of the user is close to a resting heart rate.

That the current heart rate is close to the resting heart rate means that a difference between two heart rate peaks is within a preset range (for example, a difference between the current heart rate and the resting heart rate is within 5%) or is less than a preset peak threshold, and/or change amplitudes of the two heart rates in one period are similar. For example, frequencies of the two heart rates both increase or peak values of the two heart rates both increase. The foregoing examples are merely possible manners of determining that the current heart rate is close to the resting heart rate, and are not intended to limit this embodiment of the present invention.

In addition, the resting heart rate may be a number or a number range. If the resting heart rate is a number, a heart rate within a range obtained after a number is added to or is subtracted from the number may be considered as a heart rate close to the resting heart rate. If the resting heart rate is a number range, a heart rate that falls within the number range may be considered as a resting heart rate. Optionally, a heart rate that exceeds the number range and that has a relatively small difference from an upper limit/lower limit of the number range may also be considered as a heart rate close to the resting heart rate. This is not limited herein. Alternatively, an absolute difference between the current heart rate and the resting heart rate is less than a specific value range or is less than a specific percentage of the resting heart rate. This is not limited herein.

The preset time threshold may be preset by the user based on a historical empirical value. Considering that the wearable device may incorrectly determine the state when the user just enters the stable state, when the stable state lasts for/until the preset time threshold, it may be considered that the user currently is truly in the stable state. In this case, detected stress can more effectively reflect an actual status of the user.

S416. Detect the stress of the user at a second preset time after the current heart rate of the user is close to the resting heart rate.

The second preset time may be preset by the user according to a historical empirical value. Considering that the wearable device may incorrectly determine a state, when the current heart rate of the user is close to the resting heart rate and this situation lasts for/until the second preset time, it may be considered that the user currently is truly in the stable state. In this case, the detected stress can more effectively reflect the actual status of the user.

Content in S412 to S416 is described below with reference to a specific example.

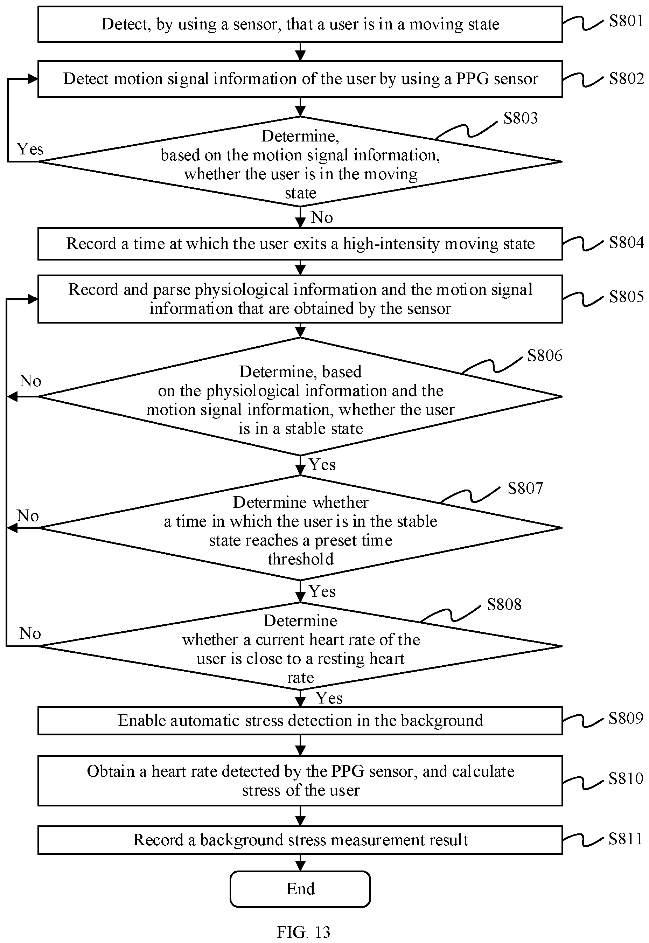

FIG. 13 shows a method procedure in which a wearable device detects stress of a user after identifying, by using a sensor, that the user enters a stable state from a moving state according to an embodiment of the present invention. The method procedure includes S801 to S811.

S801. Detect, by using the sensor, that the user is in a moving state.

For a process of determining whether the user is in the moving state, refer to the foregoing process of determining whether the user is in the sleep state. A difference lies in that a heart rate of the user in the moving state is different from a heart rate of the user in the sleep state. Likewise, an acceleration of the user in the moving state is different from an acceleration of the user in the sleep state. Details are not described herein again.

S802. Detect motion signal information of the user by using a PPG sensor.

S803. Determine, based on the motion signal information, whether the user is in the moving state. If no, S804 is performed; otherwise, the motion signal information of the user continues to be detected.

S804. Record a time at which the user exits a high-intensity moving state.

S805. Record and parse physiological information and the motion signal information that are obtained by the sensor.

S806. Determine, based on the physiological information and the motion signal information, whether the user is in a stable state. If yes, S807 is performed; otherwise, S805 is performed.

S807. Determine whether a time in which the user is in the stable state reaches a preset time threshold. If yes, S808 is performed; otherwise, S805 is performed.

S808. Determine whether a heart rate of the user is close to a resting heart rate. If yes, S809 is performed; otherwise, S805 is performed.

S809. Enable automatic stress detection in the background.

S810. Obtain a heart rate detected by the PPG sensor, and calculate the stress of the user.

S811. Record a background stress measurement result.

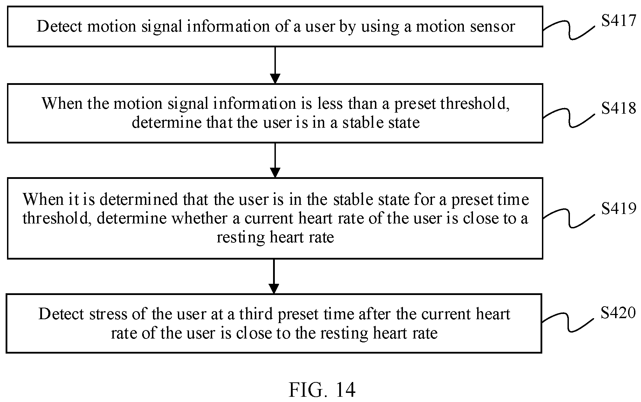

For example, as shown in FIG. 14, the detecting a status of a user by using a sensor in S304 may be specifically implemented as S417 and S418. The determining a stress measurement time based on the status of the user, and detecting stress of the user based on the time in S305 and S306 may be specifically implemented as S419 and S420.

S417. Detect motion signal information of the user by using a motion sensor.

S418. When the motion signal information is less than a preset threshold, determine that the user is in a stable state.

S419. When it is determined that the user is in the stable state for a preset time threshold, determine whether a current heart rate of the user is close to a resting heart rate.

S420. Detect the stress of the user at a third preset time after the current heart rate of the user is close to the resting heart rate.

The third preset time may be preset by the user based on a historical empirical value. Considering that the wearable device may incorrectly determine a state, when the current heart rate of the user is close to the resting heart rate and this situation lasts for/until the third preset time, it may be considered that the user currently is truly in the stable state. In this case, the detected stress can more effectively reflect an actual status of the user.

Content in S417 to S420 is described below with reference to a specific example.

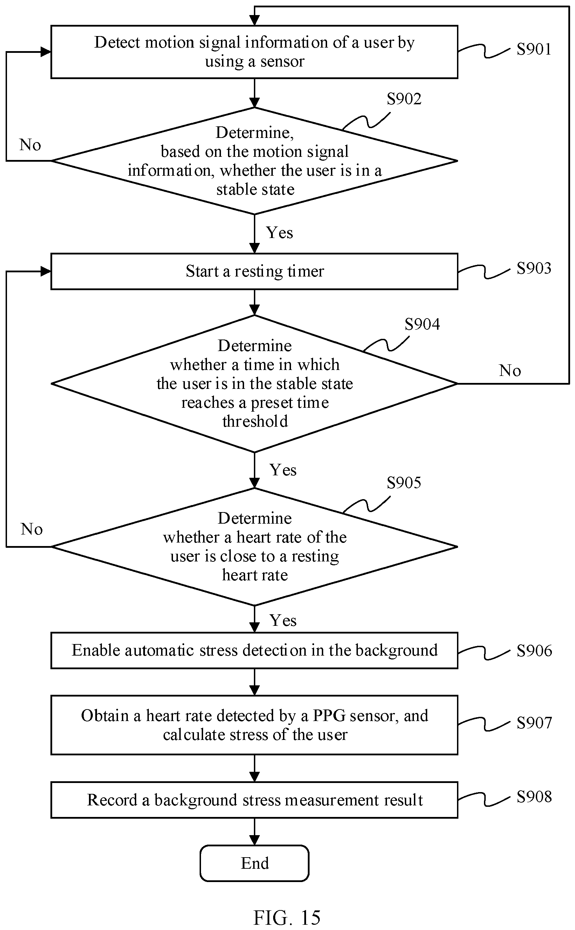

FIG. 15 shows a method procedure in which a wearable device detects stress of a user after identifying, by using a sensor, that the user is in a stable state for a long time according to an embodiment of the present invention. The method procedure includes S901 to S908.

S901. Detect motion signal information of the user by using a sensor.

S902. Determine, based on the motion signal information, whether the user is in the stable state. If yes, S903 is performed; otherwise, the motion signal information of the user continues to be detected.

For a process of determining whether the user is in the stable state, refer to the foregoing process of determining whether the user is in the sleep state. A difference lies in that a heart rate of the user in the stable state is different from a heart rate of the user in the sleep state. Likewise, an acceleration of the user in the stable state is different from an acceleration of the user in the sleep state. Details are not described herein again.

S903. Start a resting timer.

In this embodiment of the present invention, time of the resting timer may be preset by the user based on a measurement requirement of the user. The resting timer may record, through countdown, a time in which the user is in the stable state. It may be understood that the resting timer records the time in which the user is in the stable state. Therefore, any timer that can record the time in which the user is in the stable state may be understood as a resting timer.

S904. Determine whether the time in which the user is in the stable state reaches a preset time threshold. If yes, S905 is performed; otherwise, the motion signal information of the user continues to be detected.

When it is determined that the time in which the user is in the stable state exceeds the preset time threshold, to be specific, when the user is in a low-intensity moving state such as sitting for a time period, for example, has sat for one hour, in this embodiment of the present invention, it is further determined whether a current heart rate of the user is close to a resting heart rate.

S905. Determine whether the heart rate of the user is close to the resting heart rate. If yes, S906 is performed; otherwise, S903 is performed.

S906. Enable automatic stress detection in the background.

S907. Obtain a heart rate detected by the PPG sensor, and calculate the stress of the user.

S908. Record a background stress measurement result.

It should be noted that, in this embodiment of the present invention, if stress detection fails for the first time during stress measurement, on a premise that each threshold requirement is met, the stress of the user may be detected again after a delay, until the stress of the user is successfully collected.

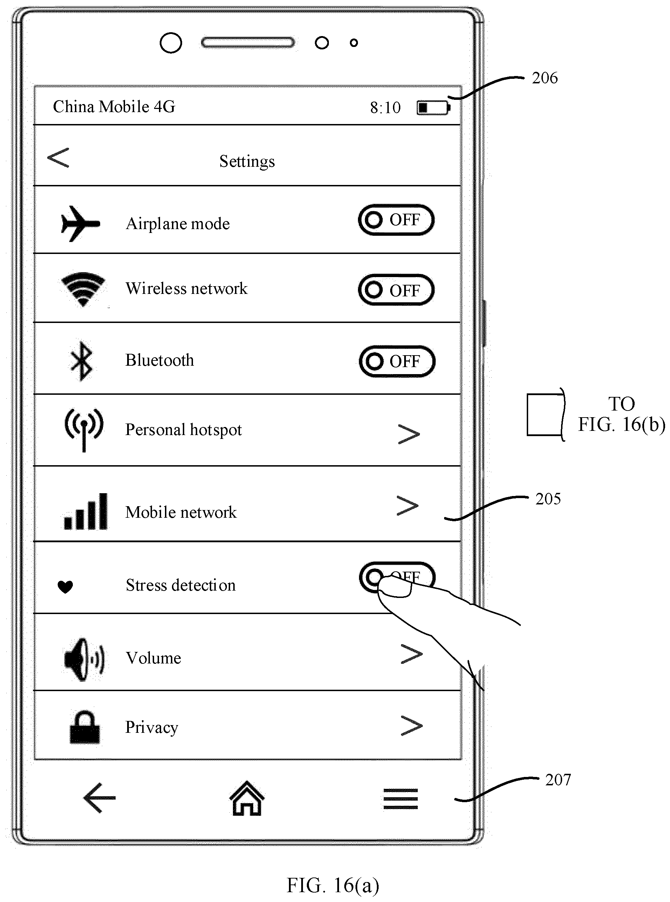

In this embodiment of the present invention, a user may preset a time for detecting stress of a user. A mobile phone is used as an example. FIG. 16(a) to FIG. 16(d) show a schematic diagram of a possible process of setting the stress detection time.

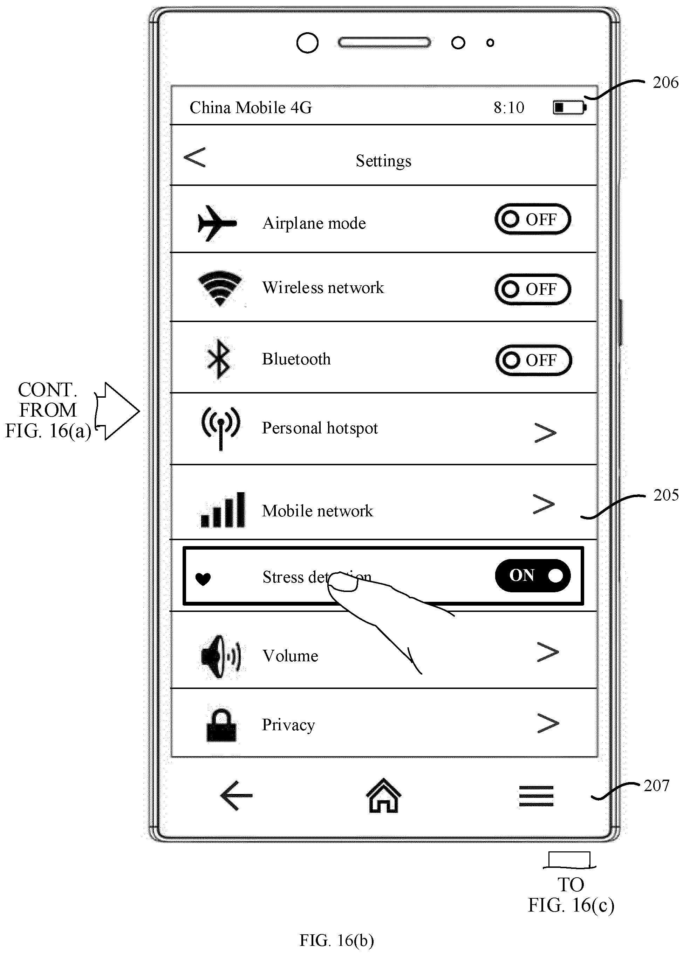

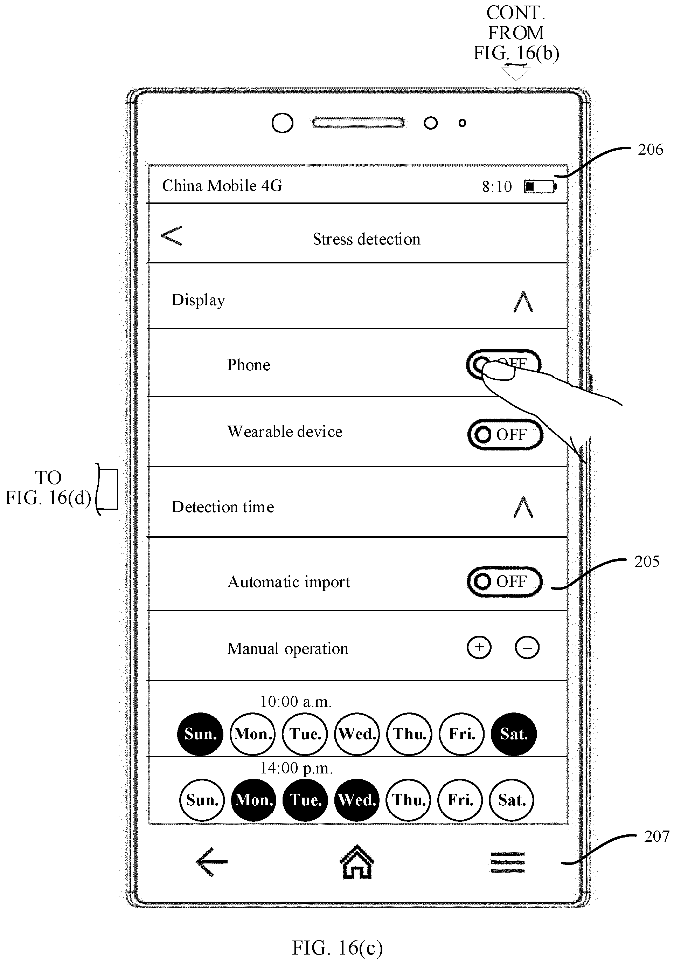

In a setting screen shown in FIG. 16(a), the user may enable a stress value measurement function through tapping. Then, a screen shown in FIG. 16(c) may be displayed by tapping an area shown in FIG. 16(b), for example, tapping stress detection. The screen includes setting options such as displaying and detection time. In the detection time option, the user may select to manually increase or decrease a detection stress time point, and/or select an automatic import manner, to directly add a time point related to a schedule or a reminder stored in the mobile phone to the detection time, or add, to the detection time, a time point randomly selected or selected according to a preset rule in a related time period. An enter manner of the detection time is not limited herein.

For example, as shown in FIG. 16(c), stress detection at a time point 10:00 a.m. is a detection process that needs to be performed every weekend, and the user may directly cancel detection on one or more days through tapping, or add detection on one or more days through tapping. Certainly, the user may directly delete the detection time point at 10:00 a.m.. This is not limited herein.

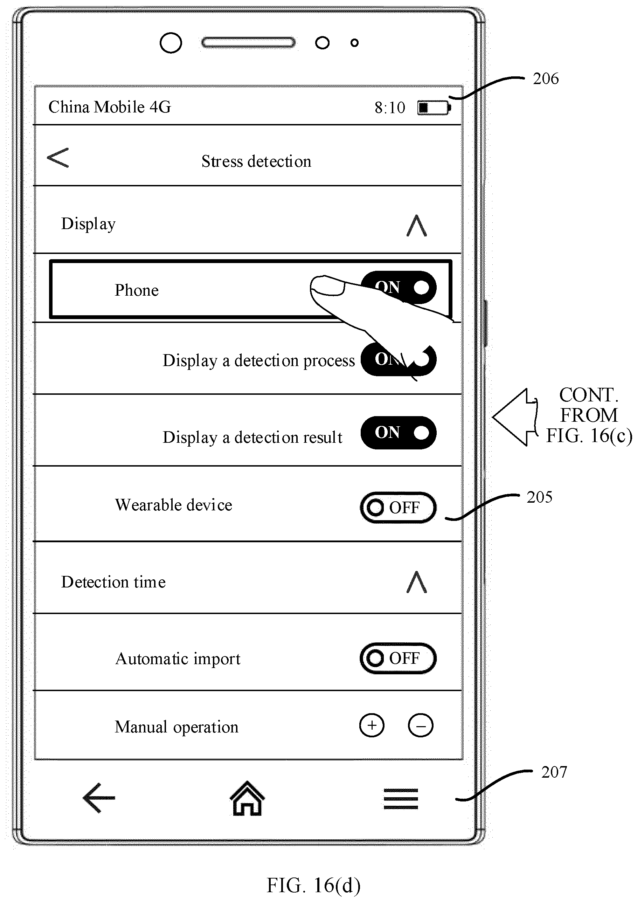

If the user chooses to enable the display function on the mobile phone, the mobile phone may switch from a currently displayed screen to a screen shown in FIG. 16(d). The user may enable, by enabling a button behind "mobile phone", options of displaying a detection process and a detection result on the mobile phone. Alternatively, the user may directly select, from a menu below the option of mobile phone, whether to display a detection process and a detection result. This is not limited herein.

A manner of displaying the detection process includes but is not limited to displaying a small icon on the top of a screen of the mobile phone in a stress detection process, and the small icon indicates that the stress of the user is currently being detected by using a wearable device and/or the terminal. Alternatively, content similar to a progress bar may be directly presented on the screen of the mobile phone, to present a current detection progress to the user. For example, data such as a percentage of the detection progress is reflected. In this embodiment of the present invention, a display result may be directly presented on the screen of the mobile phone in a form of a graph, a table, or the like, for the user to view.

It should be noted that, in a possible example, the foregoing manner of displaying the detection process and the detection result may be applied to the mobile phone, and may also be applied to a device that has a stress detection function, such as a wearable device, or a device that has a management and control relationship with a device that has a stress detection function. This is not limited herein.

In this embodiment of the present invention, detection and monitoring of the stress of a user may help the user adjust the stress of the user.

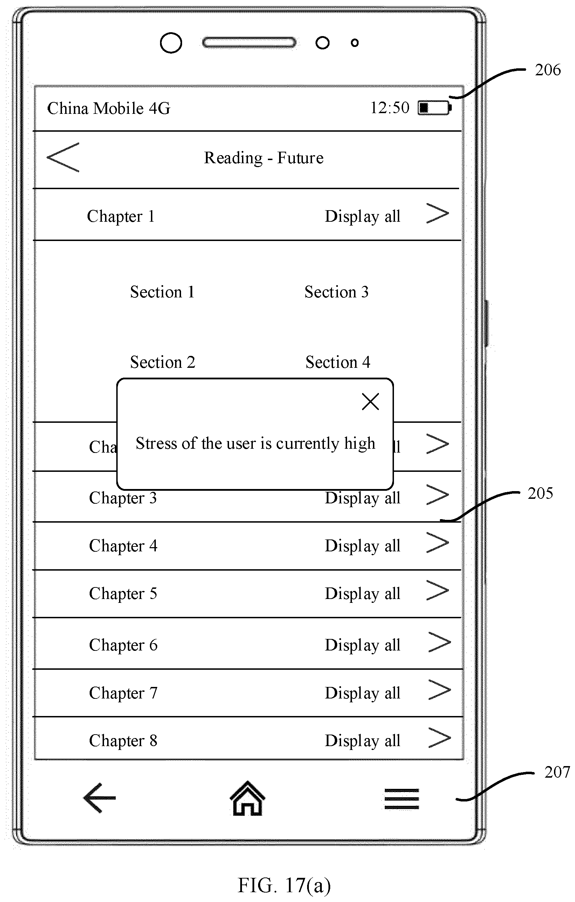

For example, when the stress of the user is relatively high, the user is prompted that the stress is currently relatively high, for example, by ringing or vibration, or in a form of a pop-up dialog box shown in FIG. 17(a). It should be noted that, in order not to affect a current operation of the user on the mobile phone, a dynamic small icon may be displayed in an area for displaying basic information of the mobile phone, to prompt the user that a message needs to be viewed, and content of the message may be "the user is currently under high stress". Alternatively, transparency of the dialog box shown in FIG. 17(a) is increased, so that the user can still clearly view content displayed on the mobile phone before the dialog box is popped up.

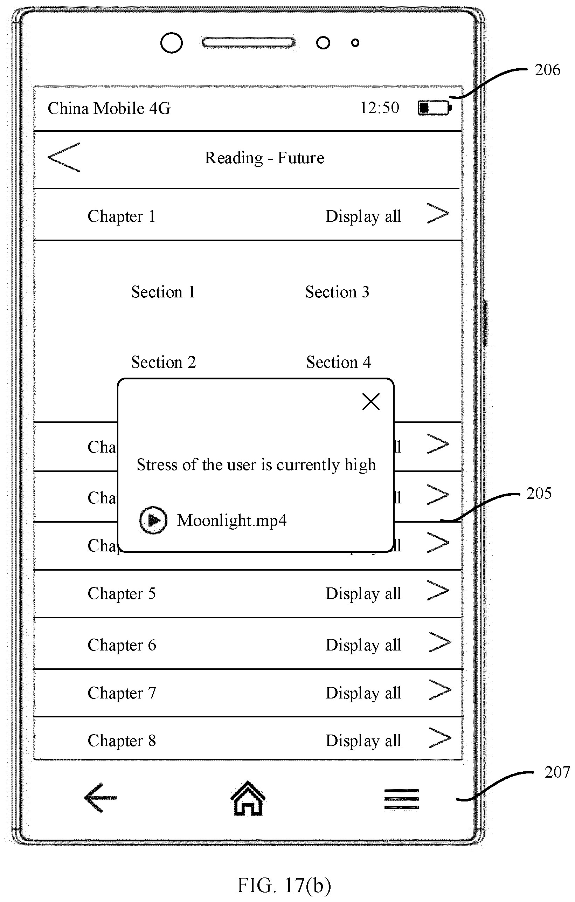

For another example, when the user is under relatively high stress, the mobile phone may present a stress adjustment scheme to the user when prompting the user that "the user is currently under relatively high stress". As shown in FIG. 17(b), the mobile phone presents, to the user, a shortcut key of an application program that can relieve stress, for example, an icon of enabling an audio and video player. Optionally, after the user taps the icon of the audio/video player, content to be played for the user may be presented to the user, for example, "Moonlight.mp4" shown in FIG. 17(b). Certainly, a list may also be provided for the user to select content to be played.

For another example, when the user is under relatively high stress, the user may be prompted to end an application program that currently causes relatively high stress to the user, or after all content presented on a current screen is automatically saved, a current application program is switched to the background or closed, so as to relieve emotion of the user, and reduce the stress of the user as quickly as possible.

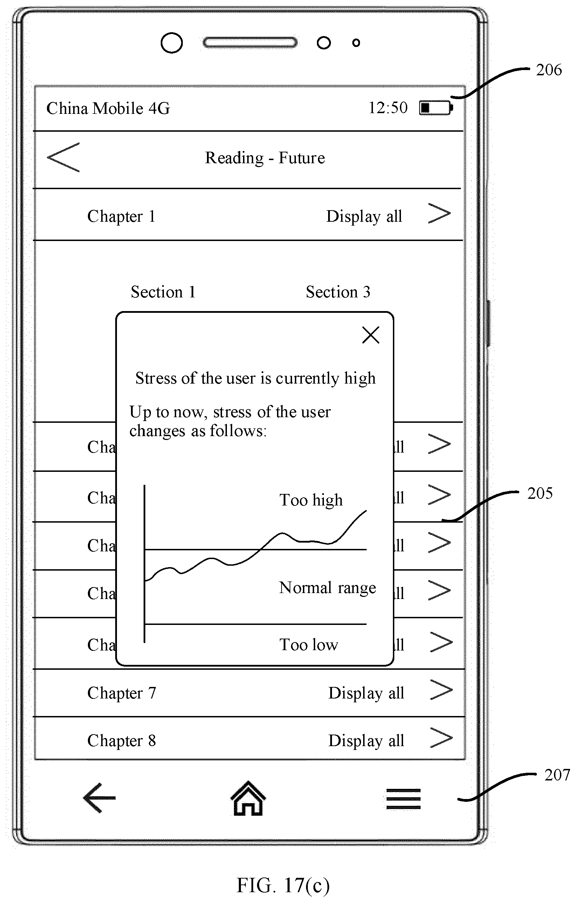

For another example, to more intuitively present a current stress status of the user to the user, the mobile phone may also present a recent change of the stress of the user to the user in a presentation form such as a curve chart or a line chart, as shown in FIG. 17(c). Certainly, to make the presentation effect more intuitive, the mobile phone may quantify the stress of the user and then present the stress of the user. The user may further be presented with a possible consequence caused if an application that currently causes a relatively high stress to the user continues to be executed, and/or the user is advised to relax in a manner such as deep breathing, and finally the user determines whether to terminate current behavior.

It should be noted that, in the foregoing implementation process, different measures may also be taken based on a source that triggers a change of stress. This is not limited herein. In addition, manners of prompting the user that the current stress is relatively high and subsequently providing an optional suggestion include but are not limited to the foregoing implementations. In addition, the mobile phone may continue to record and track the stress of the user after the prompt, or if the user expects, ignore a case in which the current user is under relatively high stress.

Similarly, the foregoing implementations are not only applicable to a scenario in which the mobile phone is independently applied, but also applicable to a scenario in which the mobile phone cooperates with the wearable device. That is, in this embodiment of the present invention, an application scenario of each of the foregoing implementation processes is not limited, and may be a scenario in which a single device or a plurality of devices work. For a specific implementation, refer to the content described above. Details are not described herein again.

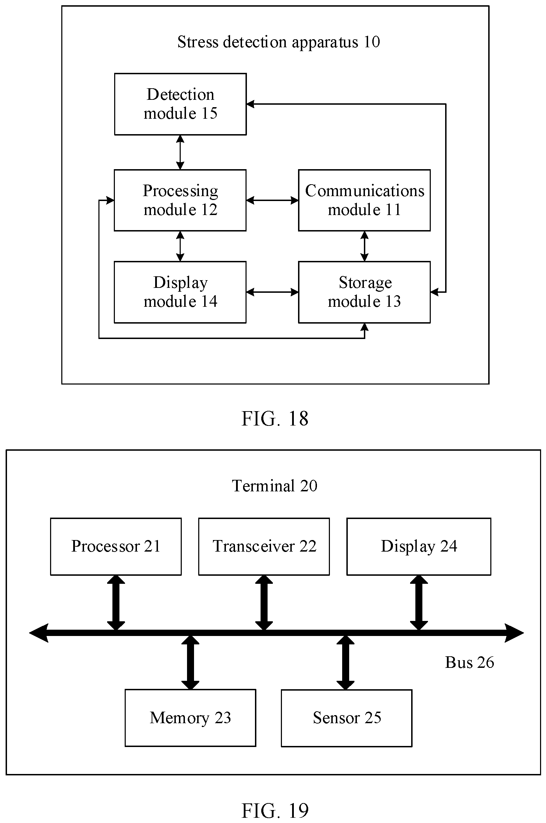

FIG. 18 is a possible schematic structural diagram of a stress detection apparatus in the foregoing embodiments. The stress detection apparatus 10 includes a communications module 11, a processing module 12, a storage module 13, a display module 14, and a detection module 15. The communications module 11 is configured to support data exchange between the stress detection apparatus 10 and each module in a terminal, for example, obtain an event such as a schedule, a reminder, or a running application program, and/or support communication between the terminal and another device such as a wearable device. The processing module 12 is configured to: support the stress detection apparatus 10 in determining an event that affects stress of a user and a stress detection time, and process data collected by the detection module 15, and/or perform another process of the technology described in this specification. The storage module 13 is configured to store program code and/or data of the terminal. The display module 14 is configured to support the stress detection apparatus 10 in presenting a stress detection process and/or a detection result to a user. The detection module 15 is configured to support the stress detection apparatus 10 in detecting a physiological signal such as a heart rate of a user.

The processing module 12 may be a processor or a controller, such as a central processing unit (Central Processing Unit, CPU), a general-purpose processor, a digital signal processor (Digital Signal Processing, DSP), an application-specific integrated circuit (Application Specific Integrated Circuit, ASIC), a field programmable gate array (Field-Programmable Gate Array, FPGA), or another programmable logic device, a transistor logic device, a hardware component, or any combination thereof. The controller/processor may implement or execute various example logical blocks, modules, and circuits described with reference to content disclosed in the present invention. The processor may be a combination of processors implementing a computing function, for example, a combination of one or more microprocessors, or a combination of the DSP and a microprocessor. The communications module 11 may be implemented as a transceiver, a transceiver circuit, a communications interface, or the like. The storage module 13 may be implemented as a memory. The display module 14 may be implemented as a display. The detection module 15 may be implemented as a sensor. For example, during stress detection of a user, the detection module 15 may be implemented as a heart rate sensor.

If the processing module 12 is implemented as an application processor, the communications module 11 is implemented as a transceiver, the storage module 13 is implemented as a memory, the display module 14 is implemented as a display, and the detection module 15 is implemented as a sensor, as shown in FIG. 19, the terminal 20 includes a processor 21, a transceiver 22, a memory 23, a display 24, a sensor 25, and a bus 26. The processor 21, the transceiver 22, the memory 23, the display 24, and the sensor 25 are connected to each other. For example, the components may be connected to each other by using the bus 26, and the bus 26 may be a peripheral component interconnect (Peripheral Component Interconnect, PCI) bus, an extended industry standard architecture (Extended Industry Standard Architecture, EISA) bus, or the like. The bus may be classified into an address bus, a data bus, a control bus, and the like. For ease of representation, a thick line is used to represent the bus in FIG. 19, but this does not mean that there is only one bus or only one type of bus.

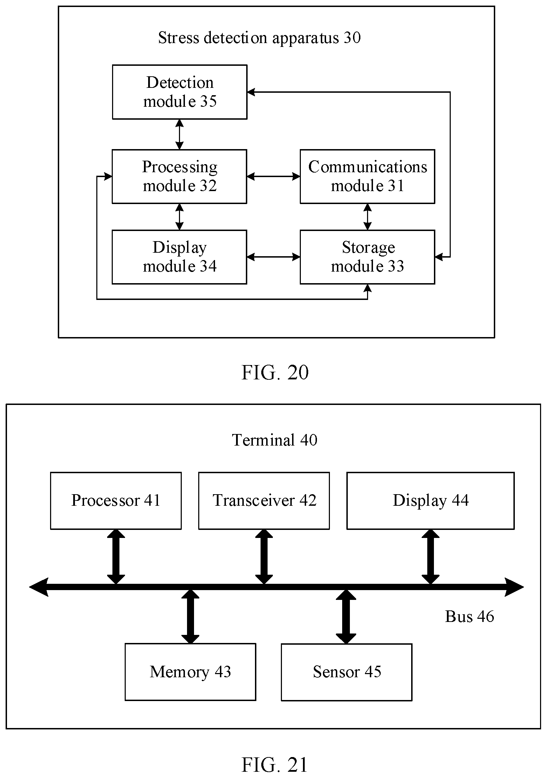

FIG. 20 is a possible schematic structural diagram of a stress detection apparatus in the foregoing embodiments. The stress detection apparatus 30 includes a communications module 31, a processing module 32, a storage module 33, a display module 34, and a detection module 35. The communications module 31 is configured to support data exchange between the stress detection apparatus 30 and each module in a terminal, and/or support communication between the terminal and another device such as a wearable device. The processing module 32 is configured to: support the stress detection apparatus 30 in determining a stress detection time, and process data collected by the detection module 35, and/or perform another process of the technology described in this specification. The storage module 33 is configured to store program code and data of the terminal. The display module 34 is configured to support the stress detection apparatus 30 in presenting a stress detection process and/or a detection result to a user. The detection module 35 is configured to support the stress detection apparatus 30 in detecting a status of a user, a physiological signal, and motion signal information.

The processing module 32 may be a processor or a controller, for example, may be a CPU, a general purpose processor, a DSP, an ASIC, an FPGA, or another programmable logic device, a transistor logic device, a hardware component, or any combination thereof. The controller/processor may implement or execute various example logical blocks, modules, and circuits described with reference to content disclosed in the present invention. The processor may be a combination of processors implementing a computing function, for example, a combination of one or more microprocessors, or a combination of the DSP and a microprocessor. The communications module 31 may be implemented as a transceiver, a transceiver circuit, a communications interface, or the like. The storage module 33 may be implemented as a memory. The display module 34 may be implemented as a display. The detection module 35 may be implemented as a sensor. For example, during detection of the status of the user, the detection module 35 may be implemented as a heart rate sensor and/or a motion sensor. During detection of the physiological signal and stress of the user, the detection module 35 may be implemented as a heart rate sensor. During detection of the motion signal information of the user, the detection module 35 may be implemented as a motion sensor.

If the processing module 32 is implemented as an application processor, the communications module 31 is implemented as a transceiver, the storage module 33 is implemented as a memory, the display module 34 is implemented as a display, and the detection module 35 is implemented as a sensor, as shown in FIG. 21, the terminal 40 includes a processor 41, a transceiver 42, a memory 43, a display 44, and a sensor 45. The processor 41, the transceiver 42, the memory 43, the display 44, and the sensor 45 are connected to each other. For example, the components may be connected to each other by using a bus 46, and the bus 46 may be a PCI bus, an EISA bus, or the like. The bus may be classified into an address bus, a data bus, a control bus, and the like. For ease of representation, a thick line is used to represent the bus in FIG. 21, but this does not mean that there is only one bus or only one type of bus.

Method or algorithm steps described in combination with the content disclosed in the present invention may be implemented by hardware, or may be implemented by a processor by executing a software instruction. The software instruction may include a corresponding software module. The software module may be stored in a flash memory, a read-only memory (Read Only Memory, ROM), an erasable programmable read-only memory (Erasable Programmable ROM, EPROM), an electrically erasable programmable read-only memory (Electrically EEPROM), a register, a hard disk, a removable hard disk, a compact disc read-only memory (Compact Disc Read-Only Memory, CD-ROM), or any other form of storage medium well known in the art. For example, a storage medium is coupled to a processor, so that the processor can read information from the storage medium or write information into the storage medium. Certainly, the storage medium may be a component of the processor. The processor and the storage medium may be deployed in a same device, or the processor and the storage medium may be deployed in different devices as discrete components. An embodiment of the present invention provides readable storage medium, including an instruction. When the instruction is run on a terminal, the terminal is enabled to perform any one of the foregoing methods. An embodiment of the present invention provides a computer program product. The computer program product includes software code, and the software code is used to perform any one of the foregoing methods. It should be noted that implementation principles of the apparatus, the terminal, the readable storage medium, and the computer program product are similar to those of the method described above. For content such as a language, a time, and an implementation, refer to the description of the method described above. Details are not described herein again.

The objectives, technical solutions, and benefits of the present invention are further described in detail in the foregoing specific embodiments. It should be understood that the foregoing descriptions are merely specific embodiments of the present invention, but are not intended to limit the protection scope of the present invention. Any modification, equivalent replacement, or improvement made within the spirit and principle of the present invention shall fall within the protection scope of the present invention.

## Claims

1. A stress detection method, wherein the method comprises:
obtaining an event of a terminal, wherein the event is at least one of a schedule, a reminder, and a running application program;
determining a stress measurement time based on the event of the terminal; and
detecting stress of a user based on the time.

2. The method according to claim 1, wherein the event is at least one of the schedule and the reminder, and the determining the stress measurement time based on the event of the terminal, and detecting stress of the user based on the time comprises:
determining, based on the schedule or the reminder, whether stress is caused to the user; and
if the schedule or the reminder causes stress to the user, detecting the stress of the user at a time point or in a time period in an occurrence process of an event in the schedule or an event in the reminder.

3. The method according to claim 1 or 2, wherein the event is the running application program, and the determining the stress measurement time based on the event of the terminal, and detecting stress of the user based on the time comprises:
determining whether the application program belongs to a first application list, wherein the first application list is a list of application programs that affect the stress of the user;
if the application program belongs to the first application list and a running time of the application program exceeds a preset time, determining whether a current heart rate of the user exceeds a resting heart rate; and
detecting the stress of the user at a time point or in a time period after the current heart rate of the user exceeds the resting heart rate.

4. A stress detection method, wherein the method comprises:
detecting a status of a user by using a sensor;
determining a stress measurement time based on the status of the user; and
detecting stress of the user based on the time.

5. The method according to claim 4, wherein the detecting the status of the user by using the sensor comprises:
detecting, by using a heart rate sensor and/or a motion sensor, that the user is in a sleep state;
detecting a physiological signal of the user by using the heart rate sensor; and
determining, based on the physiological signal, whether the user is in a waking state; and
the determining the stress measurement time based on the status of the user, and detecting stress of the user based on the time comprises:
when the user is in the waking state, obtaining a heart rate of the user by using the heart rate sensor;
determining, based on the heart rate of the user, whether the user is in a stable state; and
detecting the stress of the user at a first preset time after the user is in the stable state.

6. The method according to claim 4, wherein the detecting the status of the user by using the sensor comprises:
detecting, by using a heart rate sensor and/or a motion sensor, that the user is in a moving state;
detecting motion signal information of the user by using the motion sensor; and
determining, based on the motion signal information, whether the user is in a stable state; and
the determining the stress measurement time based on the status of the user, and detecting stress of the user based on the time comprises:
when the user is in the stable state for a preset time threshold, determining whether a current heart rate of the user is close to a resting heart rate; and
detecting the stress of the user at a second preset time after the current heart rate of the user is close to the resting heart rate.

7. The method according to claim 4, wherein the detecting the status of a user by using a sensor comprises:
detecting motion signal information of the user by using a motion sensor; and
when the motion signal information is less than a preset threshold, determining that the user is in a stable state; and
the determining a stress measurement time based on the status of the user, and detecting stress of the user based on the time comprises:
when it is determined that the user is in the stable state for a preset time threshold, determining whether a current heart rate of the user is close to a resting heart rate; and
detecting the stress of the user at a third preset time after the current heart rate of the user is close to the resting heart rate.

8. A terminal, wherein the terminal comprises a processor and a sensor;
the processor is configured to obtain an event of the terminal, wherein the event is at least one of a schedule, a reminder, and a running application program;
the processor is configured to determine a stress measurement time based on the event of the terminal; and
the sensor is configured to detect stress of a user based on the time.

9. The terminal according to claim 8, wherein the event is at least one of the schedule and the reminder, and the processor is further configured to:
determine, based on the schedule or the reminder, whether stress is caused to the user; and
if the schedule or the reminder causes stress to the user, control the sensor to detect the stress of the user at a time point or in a time period in an occurrence process of an event in the schedule or an event in the reminder.

10. The terminal according to claim 8 or 9, wherein the event is the running application program; and
the processor is further configured to:
determine whether the application program belongs to a first application list, wherein the first application list is a list of application programs that affect the stress of the user;
if the application program belongs to the first application list and a running time of the application program exceeds a preset time, determine whether a current heart rate of the user exceeds a resting heart rate; and
control the sensor to detect the stress of the user at a time point or in a time period after the current heart rate of the user exceeds the resting heart rate.

11. A terminal, wherein the terminal comprises a processor and a sensor;
the sensor is configured to detect a status of a user;
the processor is configured to determine a stress measurement time based on the status of the user that is detected by the sensor; and
the sensor is configured to detect stress of the user based on the time.

12. The terminal according to claim 11, wherein the sensor comprises a heart rate sensor and/or a motion sensor;
the heart rate sensor is configured to detect that the user is in a sleep state, and the motion sensor is configured to detect that the user is in the sleep state;
the heart rate sensor is further configured to detect a physiological signal of the user;
the processor is further configured to determine, based on the physiological signal, whether the user is in a waking state;
the heart rate sensor is further configured to: when the user is in the waking state, obtain a heart rate of the user;
the processor is further configured to determine, based on the heart rate of the user, whether the user is in a stable state; and
the sensor is further configured to detect the stress of the user at a first preset time after the user is in the stable state.

13. The terminal according to claim 11, wherein the sensor comprises a heart rate sensor and/or a motion sensor;
the heart rate sensor is configured to detect that the user is in a moving state, and the motion sensor is configured to detect that the user is in the moving state;
the motion sensor is further configured to detect motion signal information of the user;
the processor is further configured to determine, based on the motion signal information, whether the user is in a stable state;
the processor is further configured to: when the user is in the stable state for a preset time threshold, determine whether a current heart rate of the user is close to a resting heart rate; and
the sensor is further configured to detect the stress of the user at a second preset time after the current heart rate of the user is close to the resting heart rate.

14. The terminal according to claim 11, wherein the sensor comprises a motion sensor;
the motion sensor is configured to detect motion signal information of the user;
the processor is further configured to: when the motion signal information is less than a preset threshold, determine that the user is in a stable state;
the processor is further configured to: when it is determined that the user is in the stable state for a preset time threshold, determine whether a current heart rate of the user is close to a resting heart rate; and
the sensor is further configured to detect the stress of the user at a third preset time after the current heart rate of the user is close to the resting heart rate.
